# EUROPÄISCHE PATENTANMELDUNG

(11) **EP 1 512 378 A1**
(43) Veröffentlichungstag der Anmeldung: **09.03.2005**
(21) Anmeldenummer: 04029280.7
(22) Anmeldetag: 05.06.2002
(51) Int. Cl.: A61B 18/14

(54) **Elektrochirurgischer Venenstripper**

(30) Priorität: 05.06.2001 AT 8742001; 20.02.2002 AT 2592002; 23.05.2002 AT 7902002
(62) Teilanmeldung aus: 02776493.5
(71) Anmelder: E-GLOBE TECHNOLOGIES LTD., Nicosia 1101 (CY)
(72) Erfinder: Die Erfindernennung liegt noch nicht vor
(74) Vertreter: Schwarz, Albin, Dr.

(57) **Zusammenfassung**

Venenstripper mit einer Sonde und einem Koagulations- und Schneidegerät, wobei das Koagulations- und Schneidegerät zumindest zwei Elektroden und eine stimseitige Öffnung, durch die die Sonde führbar ist, aufweist, **dadurch gekennzeichnet,** daß über den im Bereich der stirnseitigen Öffnung gelegene Umfang des Koagulations- und Schneidegerätes (1) wenigstens zwei der zumindest zwei Elektroden (3,4,5) benachbart angeordnet sind.

## Beschreibung

Die Erfindung betrifft einen Venenstripper mit einer Sonde und einem Koagulations- und Schneidegerät, wobei das Koagulations- und Schneidegerät zumindest zwei Elektroden und eine stimseitige Öffnung, durch die die Sonde führbar ist, aufweist.

Venenstripper sind aus dem Bereich der Chirurgie bekannt.

Die SU 1 498 473 A1 offenbart einen Venenstripper, der mit einer Sonde verbunden ist. Der Venenzieher weist ein Ringmesser mit einem Ansatz auf, wobei im Ansatz eine Öffnung mit einer darin befindlichen Elektrode vorgesehen ist. Das Ringmesser ist als weitere Elektrode ausgebildet. Beim Venenstrippen wird das Gewebe um die Vene durch das Ringmesser geschnitten. Das Erfassen venöser Seitenäste wird durch einen erhöhten mechanischen Widerstand angezeigt. Dann kann durch Drehen des Venenziehers der venöse Seitenast mit der Schneidkante des Ansatzes durchgeschnitten und mit der Elektrode mittels Hochfrequenzströmen koaguliert werden. Nachteilig an einem Venenzieher gemäß der SU 1 498 473 A1 ist, daß kleinere venöse Seitenäste ohne merkbare Erhöhung des mechanischen Widerstandes durchtrennt und nicht koaguliert werden. Weiters ist nachteilig, daß die venösen Seitenäste nur koaguliert werden können, wenn sie kurz vor dem Durchschneiden erkannt werden. Ist ein venöser Seitenast zwar erkannt, jedoch bereits durchschnitten worden, so kann die erforderliche Lage des Venenziehers zum Koagulieren nicht mehr gefunden werden. Dies betrifft sowohl die Lage in Längsrichtung des Venenziehers als auch den einzustellenden Drehwinkel. Dabei stellt das Koagulieren eine komplizierte und langwierige Prozedur dar. Ein weiterer Nachteil des Venenziehers gemäß der SU 1 498 473 A1 ist, daß es aufgrund der Beanspruchungen beim Venenstrippen auch bei Venen im umliegenden Gewebe zu Blutungen kommt, wobei diese Venen nicht koaguliert werden. Weites ist bei einem Venenstripper gemäß der SU 1 498 473 A1 nachteilig, daß es durch die scharfe Schneidkante des Ringmessers zu unbeabsichtigten Verletzungen des umliegenden Gewebes kommen kann.

Es ist deshalb Aufgabe der vorliegenden Erfindung, einen Venenstripper der eingangs genannten Art vorzustellen, der diese Nachteile beseitigt und die Koagulation möglichst aller venösen Seitenäste gewährleistet.

Eine weitere Aufgabe der Erfindung ist es, einen Venenstripper der eingangs genannten Art anzugeben, der auch verletzte Venen im umliegenden Gewebe koaguliert.

Eine weitere Aufgabe der Erfindung ist es, einen Venenstripper der eingangs genannten Art anzugeben, bei dem die Beeinträchtigung des umliegenden Gewebes durch unbeabsichtigte Schnitte gering gehalten ist.

Weiters ist es Aufgabe der Erfindung, einen Venenstripper der eingangs genannten Art vorzustellen, der das Schneiden und Koagulieren in einem Arbeitsschritt sicherstellt.

Erfindungsgemäß wird dies dadurch erreicht, daß über den im Bereich der stimseitigen Öffnung gelegene Umfang des Koagulations- und Schneidegerätes wenigstens zwei der zumindest zwei Elektroden benachbart angeordnet sind.

Diese Ausführungsform weist den Vorteil auf, daß sie in Verbindung mit gängigen Venenstrippern verwendet werden kann. Weiters weist sie den Vorteil auf, daß ein bluttrockener Stripkanal erreicht wird, wodurch die Verwendung von Kompressionsbandagen nicht zwingend erforderlich ist. Weiters wird durch den erfindungsgemäßen Venenstripper auch die Traumatisierung des umgebenden Gewebes weitgehend vermieden. Dadurch ergibt sich der weitere Vorteil, daß die Operation mit dem erfindungsgemäßen Venenstripper das ganze Jahr hindurch und nicht nur in der kalten Jahreszeit durchgeführt werden kann.

Die Erfindung betrifft weiters einen Venenstripper mit einer Sonde und einem Koagulationsund Schneidegerät, wobei das Koagulations- und Schneidegerät zumindest eine Elektrode und eine stirnseitige Öffnung, durch die die Sonde führbar ist, aufweist.

Es ist Aufgabe der vorliegenden Erfindung, einen Venenstripper der oben genannten Art vorzustellen, der die eingangs genannten Nachteile beseitigt, die Koagulation möglichst aller venösen Seitenäste gewährleistet, der auch verletzte Venen im umliegenden Gewebe koaguliert, bei dem die Beeinträchtigung des umliegenden Gewebes durch unbeabsichtigte Schnitte gering gehalten ist, der das Schneiden und Koagulieren in einem Arbeitsschritt sicherstellt und der eine einfache und sichere Bauweise aufweist.

Erfindungsgemäß wird dies dadurch erreicht, daß die Sonde zumindest entlang eines Bereiches ihrer Länge als Elektrode ausgebildet ist.

Diese Ausführung weist den Vorteil auf, daß aufgrund der räumlichen Trennung der Elektroden die Gefahr eines Kurzschlussees besonders gering ist. Auch mit diesem erfindungsgemäßen Venenstripper wird ein bluttrockener Stripkanal erreicht, wodurch die Verwendung von Kompressionsbandagen nicht zwingend erforderlich ist. Weiters wird auch die Traumatisierung des umgebenden Gewebes weitgehend vermieden. Dadurch ergibt sich der weitere Vorteil, daß die Operation mit dem erfindungsgemäßen Venenstripper das ganze Jahr hindurch und nicht nur in der kalten Jahreszeit durchgeführt werden kann.

In Weiterbildung der Erfindung kann vorgesehen sein, daß über den im Bereich der stimseitigen Öffnung gelegene Umfang des Koagulations- und Schneidegerätes wenigstens eine der zumindest einen Elektroden des Koagulations- und Schneidegerätes angeordnet ist. Auf diese Weise kann die Koagulation in allen Richtungen um die Öffnung sichergestellt werden.

Gemäß einer weiteren Ausführungsform der Erfindung kann vorgesehen sein, daß wenigstens eine der Elektroden ringförmig ausgebildet ist. Durch die ringförmige Ausbildung kann das Umschließen der Öffnung durch die Elektrode sichergestellt werden.

Gemäß einer anderen Ausführungsform der Erfindung kann vorgesehen sein, daß wenigstens eine der Elektroden spiralförmig ausgebildet ist. Durch die spiralförmige Ausbildung kann die Öffnung mit einer Elektrode mehrfach umschlossen werden.

In weiterer Ausgestaltung der Erfindung kann vorgesehen sein, daß wenigstens eine der Elektroden Unterbrechungen aufweist. Durch die Unterbrechungen können andere Elektroden geführt werden, sodaß die Elektroden in einer Ebene angeordnet sein können.

In weiterer Ausbildung der Erfindung kann vorgesehen sein, daß wenigstens eine der Elektroden fächerförmige Finger aufweist. Durch die fächerförmigen Finger kann der Bereich, in dem koaguliert und geschnitten wird, erheblich vergrößert werden, wodurch die Sicherheit der Koagulation sämtliche venösen Seitenäste erheblich verbessert wird.

In Weiterführung der Erfindung kann vorgesehen sein, daß ein Ende der Sonde mit dem Koagulations- und Schneidegerät mittels einer am Koagulations- und Schneidegerät angeordneten Haltevorrichtung oder mittels eines Abschlusses od. dgl. verbindbar ist. Durch die Verbindung mit der Sonde kann der erfindungsgemäße Venenstripper analog einem gängigen Venenstripper verwendet werden, wodurch die Gefahr einer Fehlbedienung reduziert wird.

Gemäß einer anderen Ausführungsform der Erfindung kann vorgesehen sein, daß die Sonde mit dem Koagulations- und Schneidegerät fixiert ist. Durch die Fixierung der Sonde mit dem Koagulations- und Schneidegerät können Fehlbedienungen weitgehend ausgeschlossen werden.

Gemäß einer weiteren Ausbildung der Erfindung kann vorgesehen sein, daß das Koagulations- und Schneidegerät eine Verschiebeeinrichtung, insbesondere einen Stiel od. dgl., aufweist und daß die Sonde als Führungssonde ausgebildet ist. Durch die Verschiebeeinrichtung kann das Koagulations- und Schneidegerät über die Sonde bewegt werden, wobei die Sonde nicht mitbewegt wird und nur als Führung dient. Dadurch kann die Vene zuerst herausgeschnitten und die venösen Seitenäste koaguliert werden und erst dann die Vene herausgeholt werden. Dadurch ist es nicht erforderlich, daß das Koagulations- und Schneidegerät die Vene aufnimmt, sodaß die Abmessungen des Koagulations- und Schneidegerätes gering gehalten werden können.

In Weiterbildung der Erfindung kann vorgesehen sein, daß das Koagulations- und Schneidegerät eine Antriebsvorrichtung aufweist, wobei es selbsttätig entlang der Sonde bewegbar ist. Durch die Antriebsvorrichtung ist keine Verschiebeeinrichtung erforderlich, wodurch die Bewegung um eine gekrümmte Sonde leichter ausgeführt werden kann.

In weiterer Ausbildung der Erfindung kann vorgesehen sein, daß das Koagulations- und Schneidegerät zumindest eine weitere Elektrode, insbesondere zumindest drei weitere Elektroden, umfaßt. Durch die weiteren Elektroden kann der Bereich, in dem geschnitten und/oder koaguliert wird vergrößert werden. Dadurch wird die Sicherheit, daß alle venösen Seitenäste koaguliert werden, erhöht.

Gemäß einer weiteren Ausführungsform der Erfindung kann vorgesehen sein, daß die zumindest eine weitere Elektrode an der Wandfläche des Koagulations- und Schneidegerätes angeordnet ist. Die Anbringung der zumindest einen weiteren Elektrode an der Wandfläche des Koagulations- und Schneidegerätes erhöht den Koagulationsbereich nach dem Durchtrennen eines venösen Seitenastes und dadurch die Sicherheit, daß alle venösen Seitenäste koaguliert werden.

In Weiterführung der Erfindung kann vorgesehen sein, daß die Elektroden mit unterschiedlichen Abständen zu den jeweils benachbarten Elektroden angeordnet sind. Durch die Wahl unterschiedlicher Abstände können unterschiedliche Aktivitätsbereiche zwischen zwei Elektroden erzielt werden.

Gemäß einer anderen Ausbildung der Erfindung kann vorgesehen sein, daß zumindest eine der Elektroden mit einem Widerstand od. dgl. verbunden ist. Durch die Verbindung mit einem Widerstand od. dgl. können unterschiedliche Aktivitätsbereiche erzielt werden.

In weiterer Ausgestaltung der Erfindung kann vorgesehen sein, daß das Koagulations- und Schneidegerät einen entlang einer Längsachse ausgerichteten Grundkörper aufweist und die wenigstens zwei der zumindest zwei Elektroden an einer ersten Stirnseite des Grundkörpers angebracht sind. Am Grundkörper können die Elektroden befestigt werden. Durch die Anbringung der Elektroden an einer ersten Stirnseite wird gewährleistet, daß venöse Seitenäste jedenfalls in den Aktivitätsbereich der Elektroden gelangen bevor sie abreißen würden.

Gemäß einer weiteren Ausführungsform der Erfindung kann vorgesehen sein, daß das Koagulations- und Schneidegerät zwei im wesentlichen ringförmig ausgebildete Elektroden aufweist, wobei die Symmetrieachsen der ringförmigen Elektroden im wesentlichen parallel zueinander verlaufen und sich die von den ringförmigen Elektroden jeweils aufgespannten Flächen bei Betrachtung in Achsrichtung der Symmetrieachsen im wesentlichen überlappen. Durch diese Ausführungsform wird eine einfache Konstruktion des Koagulations- und Schneidegerätes sichergestellt, wobei es nicht erforderlich ist, die Sonde als Elektrode auszubilden.

In Weiterbildung der Erfindung kann vorgesehen sein, daß die zumindest eine weitere Elektrode ringförmig ausgebildet ist, deren Symmetrieachse im wesentlichen parallel zu den Symmetrieachsen der beiden anderen ringförmigen Elektroden verläuft, wobei sich die von der zumindest einen weiteren ringförmigen Elektrode aufgespannte Fläche und die von den beiden anderen ringförmigen Elektroden aufgespannten Flächen bei Betrachtung in Achsrichtung der Symmetrieachsen im wesentlich überlappen. Durch die Verwendung von zumindest drei ringförmigen Elektroden kann die Sicherheit der Koagulation aller venösen Seitenäste verbessert werden.

Gemäß einer anderen Ausbildung der Erfindung kann vorgesehen sein, daß die Symmetrieachsen der ringförmigen Elektroden im wesentlichen parallel zur Längsachse des Grundkörpers verlaufen. Durch diese Ausführung kann eine rotationssymmetrische Wirkung der Elektroden erreicht werden, sodaß durch ein unbeabsichtigtes Verdrehen des Koagulations- und Schneidegerätes dessen Wirkung nicht verändert wird.

Gemäß einer weiteren Ausführungsform der Erfindung kann vorgesehen sein, daß die ringförmigen Elektroden in ein und derselben, im wesentlichen normal zur Längsachse des Grundkörpers verlaufenden Ebene liegen. Durch diese Ausführungsform kann insbesondere die Schneidwirkung des Koagulations- und Schneidegerätes verbessert werden, wodurch nur eine sehr geringe Zugkraft für das Koagulieren und Schneiden benötigt wird.

In weiterer Ausgestaltung der Erfindung kann vorgesehen sein, daß der Grundkörper einen im wesentlichen zylindrischen Raum umgrenzt. Dadurch ergibt sich der Vorteil, daß das Koagulations- und Schneidegerät eng um die Vene angelegt werden kann. Weiters kann die zweite Stirnseite des Grundkörpers an einen Abschluß eines gängigen Venenstrippers angelegt werden und so mit diesem über die Vene gezogen werden, wobei venöse Seitenäste durch das Koagulations- und Schneidegerät abgetrennt werden.

In Weiterführung der Erfindung kann vorgesehen sein, daß der Grundkörper mit zumindest einem in Längsachse des Grundkörpers ausgerichteten Steg ausgebildet ist. Dies erlaubt es, daß die Vene, welche sich durch den Zug im Bereich des Grundkörpers wölbt, ausreichend ausdehnen kann, ohne den Grundkörper zu verstopfen, wodurch das Koagulations- und Schneidegerät mit einer nur geringe Länge ausgeführt werden kann.

Gemäß einer anderen Ausbildung der Erfindung kann vorgesehen sein, daß der Grundkörper im Bereich seiner zweiten Stirnseite die Haltevorrichtung zur Aufnahme und Fixierung des Endes der Sonde aufweist. Dadurch wird kein eigener Abschluß des Venenstrippers benötigt. Weiters weist diese Ausbildung den Vorteil auf, daß die Verwendung des Koagulations- und Schneidegerät in falscher Richtung ausgeschlossen ist, wodurch die Gefahr eines Fehlers bei der Bedienung reduziert wird.

Gemäß einer weiteren Ausführungsform der Erfindung kann vorgesehen sein, daß der Grundkörper an seiner zweiten Stirnseite die Haltevorrichtung zur Aufnahme und Fixierung des Endes der Sonde aufweist. Durch die Ausbildung der Haltevorrichtung an der zweiten Stirnseite kann die Baulänge des Koagulations- und Schneidegerätes verringert werden.

In weiterer Ausgestaltung der Erfindung kann vorgesehen sein, daß die Haltevorrichtung eine formschlüssige Verbindung zwischen dem Ende der Sonde und dem Grundkörper ermöglicht. Aufgrund der formschlüssigen Ausführung der Verbindung wird sichergestellt, daß sich die Verbindung nicht unbeabsichtigt gelöst wird.

In Weiterführung der Erfindung kann vorgesehen sein, daß die Haltevorrichtung einen in der Seitenwand des Grundkörpers angebrachten Schlitz zum Einlegen der Sonde sowie eine Vertiefung zur Aufnahme des Endes der Sonde umfaßt, wobei die Vertiefung an Ihrer der ersten Stirnseite des Grundkörpers zugewandten Seite einen diese verengenden Steg aufweist. Diese Ausführung ermöglicht eine einfache und sichere Verbindung zwischen der Sonde und dem Grundkörper.

Gemäß einer weiteren Ausführungsform der Erfindung kann vorgesehen sein, daß die Vertiefung an Ihrer der ersten Stirnseite des Grundkörpers zugewandten Seite eine Verlängerung aufweist. Durch die Verlängerung kann die Dauerhaftigkeit der Verbindung verbessert werden.

In weiterer Ausbildung der Erfindung kann vorgesehen sein, daß elektrische Leitungen zur Verbindung der Elektroden mit einer Stromversorgungsquelle zentrisch an der zweiten Stirnseite des Grundkörpers aus dem Grundkörper herausgeführt sind. Aufgrund dieser Ausführung kann das Koagulations- und Schneidegerät mittels der elektrischen Leitungen ohne Kippmoment bewegt werden.

Gemäß einer anderen Ausgestaltung der Erfindung kann vorgesehen sein, daß die Haltevorrichtung zwei Kontakte aufweist, die mit den Elektroden elektrisch leitend verbunden sind und daß die elektrischen Leitungen zur Verbindung der Elektroden mit einer Stromversorgungsquelle innerhalb der Sonde geführt und mit an dem Ende der Sonde liegenden weiteren Kontakten verbunden sind, wobei die elektrisch leitende Verbindung zwischen den ringförmigen Elektroden und der Stromversorgungsquelle bei der Aufnahme und Fixierung der Sonde in der Haltevorrichtung durch die Kontakte und die weiteren Kontakte erfolgt. Somit müssen keine eigenen Kabel zur Verbindung der Elektroden mit der Stromversorgungsquelle vorgesehen werden, da die Verbindung über die Sonde erfolgt.

In Weiterbildung der Erfindung kann vorgesehen sein, daß ein Drainageschlauch am Koagulations- und Schneide gerät angeordnet ist. Auf diese Weise kann der Drainageschlauch in einem Arbeitsgang mit dem Strippen in den Stripkanal eingebracht werden.

In diesem Zusammenhang kann in Weiterführung der Erfindung vorgesehen sein, daß an der der Öffnung gegenüberliegenden Stirnseite des Koagulations- und Schneidegerätes eine Drainageschlauch-Befestigungsvorrichtung angeordnet ist. Bei der Verwendung des erfindungsgemäßen Venenstrippers für zwei Venenstücke kann der nach dem ersten Venenstück im Stripkanal befindliche Drainageschlauch abgeschnitten und ein weiterer Drainageschlauch erneut am Koagulations- und Schneidegerät befestigt werden.

Gemäß einer anderen Ausführungsform der Erfindung kann vorgesehen sein, daß eine Längs-Schlitzvorrichtung vorgesehen ist. Durch die Längs-Schlitzvorrichtung kann der erfindungsgemäße Venenstripper um die Vene geführt werden, wobei die Vene erst nach dem Venenstrippen aus dem Stripkanal entfernt wird. Dadurch ergibt sich der Vorteil, daß die Vene während des Strippens in gestreckten Lage verbleiben kann.

Gemäß einer weiteren Ausgestaltung der Erfindung kann vorgesehen sein, daß die Längs-Schlitzvorrichtung eine Schlitzelektrode zum monopolaren Schneiden, eine Schlitzelektrode zum bipolaren Schneiden oder eine mechanische Schneide umfaßt. Durch jede dieser drei Ausführungsfbrmen kann ein sicheres Aufschlitzen der Vene in Längsrichtung sichergestellt werden.

In Weiterführung der Erfindung kann vorgesehen sein, daß die Längs-Schlitzvorrichtung mit dem Koagulations- und Schneidegerät verbunden ist, und daß gegebenenfalls die Schlitzelektrode mit den elektrischen Leitungen oder einer weiteren elektrischen Leitung verbunden ist. Auf diese Weise ist die Längs-Schlitzvorrichtung auf einfache Weise herstellbar.

Gemäß einer weiteren Ausführungsform der Erfindung kann vorgesehen sein, daß die Längs-Schlitzvorrichtung mit der Sonde, insbesondere mit einer Nut od. dgl. der Sonde, verrastbar ist. Mit dieser Ausführungsform kann das sichere Schlitzen der Vene in Längsrichtung gewährleistet werden.

In weiterer Ausgestaltung der Erfindung kann vorgesehen sein, daß die Längs-Schlitzvorrichtung in der Haltevorrichtung fixiert werden kann und eine weitere Haltevorrichtung zur Aufnahme und Fixierung des Endes der Sonde aufweist. Diese Ausgestaltung ermöglicht einen modularen Aufbau des Koagulations- und Schneidegerätes und der Längs-Schlitzvorrichtung.

In Weiterführung der Erfindung kann vorgesehen sein, daß im Bereich wenigstens einer der Elektroden wenigstens eine Auslaßöffnung für den Austritt eines ionisierbaren Gases, insbesondere Argon, vorgesehen ist. Durch das ionisierbare Gas tritt bereits vor den Elektroden eine Koagulation auf, wobei die Gefahr, daß Verkrustungen od. dgl. an einer der Elektroden anhaften und bei der Bewegung des Venenstrippers aufgerissen werden, verringert wird. Das ionisierbare Gas wird über eine Gaszuleitung zugeführt.

In diesem Zusammenhang kann gemäß einer weiteren Ausführungsform der Erfindung vorgesehen sein, daß die wenigstens eine Auslaßöffnung mit einem Gasanschluß an der zweiten Stirnseite des Grundkörpers verbunden ist. Dabei kann die Gaszuleitung und die elektrischen Leitungen parallel und gegebenenfalls in einem gemeinsamen Schlauch geführt werden.

In weiterer Ausgestaltung der Erfindung kann vorgesehen sein, daß zumindest eine Absaugöffnung vorgesehen ist. Mittels der Absaugöffnung kann der beim Koagulieren entstehende Rauch abgesaugt und über eine Absaugleitung abgeführt werden.

In diesem Zusammenhang kann in Weiterführung der Erfindung vorgesehen sein, daß die zumindest eine Absaugöffnung in der Haltevorrichtung angeordnet ist. Durch diese Ausbildung kann die Absaugöffnung unabhängig von dem Koagulations- und Schneidegerät ausgebildet werden, wobei eine gute Absaugung sichergestellt werden kann.

In weiterer Ausgestaltung der Erfindung kann vorgesehen sein, daß die zumindest eine Absaugöffnung mit einem Absauganschluß an der zweiten Stirnseite des Grundkörpers verbunden ist. Dadurch können die elektrischen Leitungen, die Absaugleitung und gegebenenfalls die Gaszuleitung parallel geführt werden und gegebenenfalls in einem gemeinsamen Schlauch angeordnet werden.

In Weiterführung der Erfindung kann vorgesehen sein, daß im Bereich der Elektroden ein ringförmiger Ultraschallresonator angeordnet ist. Dabei kann der Ultraschallresonator als Schneidegerät und die Elektroden als Koagulationsgerät ausgebildet sein, wodurch der Schneidevorgang und der Koagulationsvorgang getrennt sind und auf einfache Weise unabhängig voneinander eingestellt und geregelt werden können.

Die Erfindung wird unter Bezugnahme auf die beigeschlossenen Zeichnungen, in welchen besonders bevorzugte Ausführungsbeispiele dargestellt sind, näher beschrieben. Dabei zeigt:
Fig. 1a eine Vene mit Seitenästen im Bindegewebe im Schnitt;
Fig. 1b die freigelegte Vene nach Fig. 1a mit durchgeführter Sonde und Abschluß und Griff im Schnitt;
Fig. 1c die Vene nach Fig. 1a mit Aufwölbungen beim Strippvorgang im Schnitt;
Fig. 2a eine Vene im Bindegewebe mit übergestülpten Elektroden im Schnitt;
Fig. 2b eine Darstellung einer Anordnung der Elektroden gegenüber der Vene;
Fig. 3a eine Anordnung der Elektroden in zwei unterschiedlichen Ebenen im Seitenriß;
Fig. 3b eine weitere Anordnung der Elektroden in zwei unterschiedlichen Ebenen im Seitenriß;
Fig. 3c eine Anordnung der Elektroden in derselben Ebene im Seitenriß;
Fig. 3d eine weitere mögliche Anordnung der Elektroden im Aufriß;
Fig. 4a ein Koagulations- und Schneidegerät eines erfindungsgemäßen Venenstrippers im Seitenriß;
Fig. 4b einen erfindungsgemäßen Venenstripper, wobei das Koagulations- und Schneidegerät einen zylinderförmigen Grundkörper aufweist, im Seitenriß;
Fig. 4c einen erfindungsgemäßen Venenstripper wobei das Koagulations- und Schneidegerät zwei Stege aufweist, im Seitenriß;
Fig. 4d einen erfindungsgemäßen Venenstripper wobei das Koagulations- und Schneidegerät zwei Stege und eine Haltevorrichtung aufweist, im Seitenriß;
Fig. 4e einen Schnitt der Haltevorrichtung des Koagulations- und Schneidegerätes und der Sonde gemäß Fig. 4d;
Fig. 4f einen erfindungsgemäßen Venenstripper, wobei das Koagulations- und Schneidegerät eine Haltevorrichtung mit Kontakten aufweist und in der Sonde elektrische Leitungen geführten sind, sowie einen mit Kontakten versehenen Griff, im Schnitt;
Fig. 4g einen erfindungsgemäßen Venenstripper, wobei das Koagulations- und Schneidegerät eine Verschiebeeinrichtung aufweist;
Fig. 4h einen erfindungsgemäßen Venenstripper, wobei das Koagulations- und Schneidegerät eine Antriebsvorrichtung aufweist;
Fig. 4i einen erfindungsgemäßen Venenstripper, wobei das Koagulations- und Schneidegerät eine Verschiebeeinrichtung aufweist und die Sonde als Elektrode ausgebildet ist;
Fig. 4j einen erfindungsgemäßen Venenstripper, wobei die Sonde als Elektrode ausgebildet ist;
Fig. 4k einen erfindungsgemäßen Venenstripper, wobei das Koagulations- und Schneidegerät eine Antriebsvorrichtung aufweist und die Sonde als Elektrode ausgebildet ist;
Fig.5a ein weiteres Koagulations- und Schneidegerät eines erfindungsgemäßen Venenstrippers teilweise im Schnitt;
Fig. 5b die Seitenansicht des Koagulations- und Schneidegerätes gemäß Fig. 5a, im Schnitt;
Fig. 5c die Draufsicht des Koagulations- und Schneidegerät gemäß Fig. 5a;
Fig. 5d die Vorderansicht des Koagulations- und Schneidegerät gemäß Fig. 5a;
Fig. 5e die Seitenansicht einer weiteren Ausführungsform des Koagulations- und
Schneidegerätes mit einer Längs-Schlitzvorrichtung, im Schnitt;
Fig. 5f die Seitenansicht einer weiteren Ausführungsform des Koagulations- und
Schneidegerätes mit einer Längs-Schlitzvorrichtung und einer weiteren Haltevorrichtung, im Schnitt;
Fig. 6a eine erfindungsgemäße Ausgestaltung der Elektroden mit abschnittsweisen Unterbrechungen.
Fig. 6b eine andere erfindungsgemäße Ausgestaltung der Elektroden, welche spiralförmig ausgebildet sind;
Fig. 6c eine weitere erfindungsgemäße Ausgestaltung der Elektroden, welche fächerförmige Finger aufweisen;
Fig. 7 eine Schrägansicht einer weiteren Ausführungsform eines erfindungsgemäßen Venenstrippers mit Auslaßöffnungen;
Fig. 8 eine Schrägansicht einer anderen Ausführungsform eines erfindungsgemäßen Venenstrippers mit Absaugöffnungen;
Fig. 9a eine Schrägansicht einer weiteren Ausführungsform eines erfindungsgemäßen Venenstrippers mit Auslaßöffnungen und Absaugöffnungen.
Fig. 9b eine Seitenansicht des Venenstrippers gemäß Fig. 9a;
Fig. 9c eine Draufsicht auf den Venenstripper gemäß Fig. 9a;
Fig. 9d eine Schnittansicht entlang der Linie AA in Fig. 9b;
Fig. 9e eine Schnittansicht entlang der Linie BB in Fig. 9c; und
Fig. 10 eine Schrägansicht einer anderen Ausführungsform eines erfindungsgemäßen Venenstrippers mit einem Ultraschallresonator und Absaugöffnungen.

Die vorliegende Erfindung steht im Zusammenhang mit Techniken der Elektrochirurgie bzw. Hochfrequenz-Chirurgie und insbesondere mit dem Koagulieren bzw. dem Schneiden biologischen Materials oder Gewebes mittels hochfrequentem, durch das Gewebe fließenden Stromes. Die Effekte auf das biologische Material bei niederfrequenten Strömen sind der elektrolytische Effekt und der faradayische Effekt. Diese beiden Effekte sind aber in der Elektrochirurgie nicht erwünscht, da sie einerseits zur elektrolytischen Schädigungen und andererseits zu ungewollten Muskelkontraktionen führen können. Aus diesem Grund werden in der Elektrochirurgie ausschließlich hochfrequente Ströme mit Frequenzen ab etwa 300kHz verwendet. Bei diesen Frequenzen erfolgt keine Ionenverschiebung im Gewebe und es treten auch keine Muskelkontraktionen mehr auf. Man erreicht ein Erhitzen des Gewebes durch den thermischen Effekt. Dieser kann zum Koagulieren oder zum Schneiden des Gewebes benutzt werden.

Beim Koagulieren, bei dem das Gewebe auf etwa 100°C erhitzt wird, verdampft intra- und extrazelluläre Flüssigkeit. Dadurch schrumpft das Gewebe, wobei die Zellmembranen aber intakt bleiben. Auf diese Weise lassen sich Blutungen mit großem Erfolg stillen. Beim Schneiden wird das Gewebe schlagartig auf Temperaturen, welche etwas oberhalb von 100°C liegen, erhitzt, sodaß die Zellmembranen explosionsartig zerreißen. Dies ermöglicht es, exakte Schnitte im Gewebe durchzuführen.

Aus der Ausformung der Elektroden zur Verbindung mit der hochfrequenten Stromversorgungsquelle ergeben sich im wesentlichen zwei Techniken der Elektrochirurgie. Bei der monopolaren Technik wird eine großflächige sogenannte neutrale Elektrode am Körper des Patienten angebracht. Die Größe der Elektrode bewirkt, daß in diesem Bereich nur geringe Stromdichten auftreten, weswegen sich nur geringe bzw. vernachlässigbare Erwärmungen ergeben. Die Erwärmung erfolgt im Bereich der aktiven Elektrode, welche spitz ausgeführt ist und in diesem Bereich erhebliche Stromdichten im Gewebe hervorruft. Bei der bipolaren Technik sind beide Elektroden sehr nahe beieinander gelegen und möglichst in einem Gerät integriert. Dabei fließt der Strom nur in dem eng umschriebenen Gewebebereich zwischen den beiden Elektroden.

Der erfindungsgemäße Venenstripper bezieht sich auf die bipolare Technik zum Koagulieren bzw. Schneiden von organischen Material.

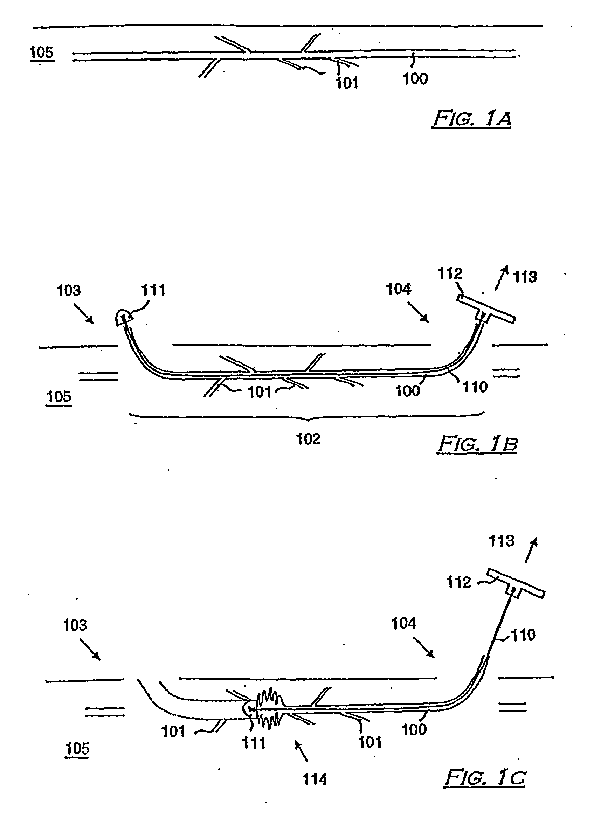

Bei Gefäß- bzw. Venenerkrankungen ist es sehr häufig notwendig, einen bestimmten Abschnitt 102 einer Vene 100 zu entfernen (Fig. 1a-1c).

Hierfür wird das Bindegewebe 105 an den den Abschnitt 102 begrenzenden oberen, d.h. Herz-nahen Bereich 103 und unteren, d.h. Herz-fernen Bereich 104 eingeschnitten, die Vene 100 durchtrennt, und die beiden Enden des zu entfernenden Abschnittes 102 der Vene 100 freigelegt.

In einem nächsten Schritt wird die Sonde 110 eines Venenstrippers in den zu entfernenden Abschnitt 102 der Vene 100 eingeführt.

Auf die Sonde 110 wird im unteren Bereich 103 ein Abschluß 111 angebracht, der ein Abrutschen der Vene 100 von der Sonde 110 verhindert. Anschließend wird die Sonde 110 mittels eines am oberen Ende der Sonde 110 angebrachten Griffs 112 durch des Bindegewebe 105 unter Mitnahme des zu entfernenden Abschnitts 102 hindurch- und im oberen Bereich 103 in der durch den Pfeil 113 angedeuteten Zugrichtung herausgezogen.

Wie in Fig. 1c angedeutet, kommt es dabei in dem dem Abschluß 111 vorgelagerten Bereich 114 zu einer Aufwölbung der Vene 100 bzw. zu verstärkter Faltenbildung in der Venenwand.

Nachteilig bei diesem Verfahren ist, daß die venösen Seitenäste 101, welche in die Vene 100 einmünden, bei diesem Vorgang abgerissen werden. Dies verursacht beträchtliche Blutungen und bedingt längere Krankenhausaufenthalte bei entsprechender Bandagierung und Ruhigstellung des behandelten Körperteiles.

Der erfindungsgemäße Venenstripper erlaubt es, diesen Nachteil zu beseitigen. Dies wird dadurch erreicht, daß die venösen Seitenäste 101 nicht mit der Vene 100 aus dem Bindegewebe 105 herausgerissen bzw. beim Herausziehen des Venen-Abschnittes 102 abgerissen werden, sondern vorher mit dem vorgestellten Gerät verschlossen und von der Vene 100 getrennt werden.

Dies erfolgt durch das weiter oben beschriebene elektrochirurgische Koagulieren oder Schneiden der venösen Seitenäste 101 im Bereich deren Einmündung in die Vene 100.

Dafür umfaßt der erfindungsgemäße Venenstripper ein Koagulations- und Schneidegerät 1, welches gemäß einer ersten Ausführungsform des erfindungsgemäßen Venenstrippers mit zumindest zwei Elektroden 3, 4, 5 ausgebildet ist, welche im wesentlichen ringförmig sein können. Gemäß einer anderen Ausführungsform des erfindungsgemäßen Venenstrippers ist die Sonde 110 als Elektrode 3, 4, 5 und das Koagulations- und Schneidegerät 1 mit zumindest einer Elektrode 3, 4, 5 ausgebildet, wobei der erfindungsgemäße Venenstripper wieder zumindest zwei Elektroden 3, 4, 5 aufweist.

Durch die ringförmige Ausführung der Elektroden 3, 4, 5 können diese über die Vene 100 gestülpt werden.

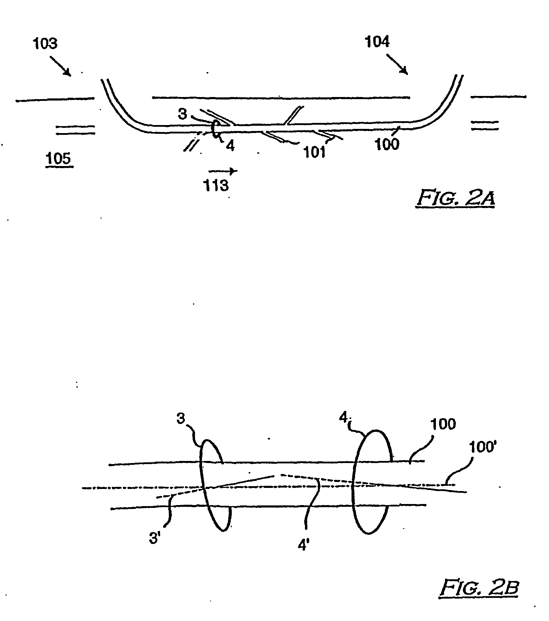

Dies erzeugt einen im allgemeinen ringförmigen, die Vene 100 in sich einschließenden Bereich -im folgenden Aktivitätsbereich 6 genannt-, in dem das die Vene 100 umschließende biologische Material koaguliert bzw. geschnitten werden kann. Somit können die venösen Seitenäste 101 im Bereich ihrer Einmündung in die Vene 100 von der Vene 100 getrennt werden (Fig. 2). Es ist dabei einerseits möglich, die venösen Seitenäste 101 unmittelbar zu durchschneiden. Andererseits ist es auch ausreichend, ein Koagulieren der venösen Seitenäste 101 zu bewirken. Diese werden dadurch verschlossen und werden an der koagulierten Stelle beim nachfolgenden Herausziehen des zu entfernenden Abschnittes 102 der Vene 100 sauber getrennt, ohne daß es zu nennenswerten Blutungen kommt.

Der optimale Radius der Elektroden 3, 4, 5 richtet sich nach den physiologischen Gegebenheiten. Diese sind bestimmt durch den Durchmesser der Vene 100. Andererseits ist der Durchmesser der Elektroden 3, 4, 5 möglichst klein zu halten, damit möglichst wenig umstehendes Bindegewebe 105 verletzt wird. In diesem Sinn ist die ringförmige Ausbildung der Elektroden 3,4, 5 beim Venenstripping besonders vorteilhaft, da die Elektroden 3,4, 5 hier besonders eng an die Vene 100 anlegt werden können. Es sind aber auch andere Ausbildungen der Elektroden 3, 4, 5, beispielsweise als geschlossene Schleifen mit rechteckigen Grundriß etc. denkbar. Auch diese Elektroden 3, 4, 5 gelten als im wesentlichen ringförmige Elektroden 3, 4, 5 im Sinne der Erfindung.

Für das Überstülpen der im wesentlichen ringförmigen Elektroden 3, 4, 5 über die Vene 100, werden diese so angeordnet, daß die Symmetrieachsen 3', 4', 5' der Elektroden 3, 4, 5 im wesentlichen parallel zur Mittellinie 100' der Vene 100 verlaufen (Fig. 2b). Die Symmetrieachsen 3', 4', 5' der Elektroden 3, 4, 5 verlaufen somit auch im wesentlichen parallel zueinander. Weiters überlappen sich die von den Elektroden 3, 4, 5 jeweils aufgespannten Flächen, wenn diese in Achsrichtung der Symmetrieachsen 3', 4', 5' betrachtet werden. Diese Überlappung kann vollständig oder auch nur teilweise, je nach Ausführung der einzelnen Elektroden 3, 4, 5 sein.

Dies gilt natürlich auch für Elektroden 3, 4, 5, bei denen keine exakte Symmetrieachse 3', 4', 5' definiert werden kann, die also beispielsweise Ausbuchtungen oder ähnliches aufweisen.

Die Anordnung der im wesentlichen ringförmigen Elektroden 3, 4, 5 kann aber in jedem Fall sinngemäß gemäß der in Fig. 2b skizzierten Anordnung erfolgen.

Die genaue Anordnung der Elektroden 3,4, 5 beeinflußt die Position und Ausdehnung des Aktivitätsbereichs 6, d.h. jenes Bereichs, in welchem die thermischen Effekte auf das Gewebe am größten sind. Der Aktivitätsbereich 6 ist durch jenen Bereich umrissen, in welchem die Stromdichte zwischen den Elektroden 3, 4, 5 am größten ist.

Form und Struktur des Aktivitätsbereichs 6 sind natürlich auch von anderen Faktoren, insbesondere von den verwendeten Strom- und Spannungsstärken abhängig. So können über die Wahl der Ausgangsspannungen unterschiedliche Methoden der Koagulation wie Soft Koagulation, Forced Koagulation und Spray Koagulation eingestellt werden.

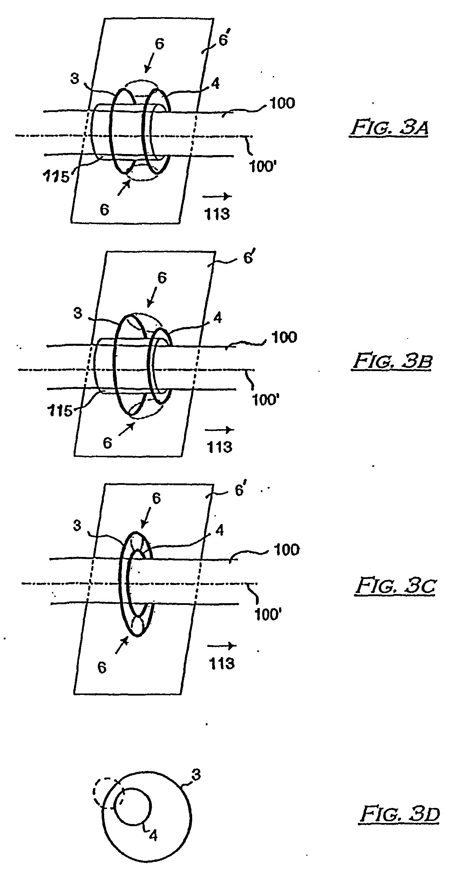

Die Fig. 3a bis 3d zeigen einige mögliche vorteilhafte Anordnungen der Elektroden 3, 4, 5 sowie die sich aus diesen Anordnungen ergebenden Aktivitätsbereiche 6.

Die Aktivitätsbereiche 6 sind dabei jeweils durch gestrichelte Linien angedeutet. Diese ergeben sich als Schnittlinien zwischen der Randfläche des dreidimensionalen im wesentlichen ringförmigen Aktivitätsbereichs 6 und der Zeichenebene, welche durch die Ebene 6' angedeutet ist. Der durch die ringförmigen Elektroden 3, 4, 5 definierte Aktivitätsbereich 6 ist aber in jedem Fall ein dreidimensionales etwa ringförmiges Gebiet. Allgemein kann der Aktivitätsbereich 6 als Torus definiert werden, mit kreisförmigen oder auch elliptischen bzw. rechteckigen Grundriß.

Fig. 3a zeigt eine Anordnung, bei der zwei Elektroden 3, 4 in zwei unterschiedlichen Ebenen liegen, welche beabstandet zueinander und im wesentlichen parallel sind. Dies ergibt einen Aktivitätsbereich 6 für das bipolare Koagulieren bzw. Schneiden, welcher wie dargestellt zwischen den beiden Elektroden 3, 4 liegt. Mit dieser Anordnung wird bei der Bewegung der beiden Elektroden 3, 4 in Zugrichtung 113 ein venöser Seitenast 101 zunächst durch die Elektrode 4 erfaßt. Erst bei weiterem Zug wird der Seitenast 101 in den Aktivitätsbereich 6 gebogen und hier koaguliert bzw. geschnitten.

In diesem Fall ist es zweckmäßig, eine eigene Schutzhülle 115 vorzusehen, welche verhindert, daß die Vene 100 selbst durchschnitten wird.

Fig. 3b zeigt eine Anordnung mit zwei Elektroden 3, 4, 5 mit unterschiedlichen Durchmessern, die wieder in zwei beabstandeten Ebenen liegen. Der skizzierte Aktivitätsbereich 6 führt schon früher, d.h. bei geringerem Zug, zum Schneiden bzw. Koagulieren der venösen Seitenäste 101. Auch hier kann eine Schutzhülle 115 vorgesehen sein.

Bei der in Fig. 3c skizzierten Anordnung liegen die zwei Elektroden 3, 4 in der selben Ebene. Dadurch erhält man einen Aktivitätsbereich 6, der sich nicht wesentlich über den durch die innere Elektrode 4 und die äußere Elektrode 3 gebildeten Rand erstreckt. Damit liegt die Vene 100 selbst nicht im Aktivitätsbereich 6. Bei der Bewegung in Zugrichtung 113 werden venöse Seitenäste 101 bereits vor und vor allem bei der Berührung mit einer der beiden Elektroden 3, 4 durchtrennt. Dies erleichtert das Schneiden nach vorne, da der Aktivitätsbereich in Zugrichtung 113 liegt. Eine Schutzhülle 115 ist hier nicht notwendig, dennoch kann durch die Halterung der beiden Elektroden 3, 4 sichergestellt werden, daß auch die innere Elektrode 4 nicht zu nahe an die Vene 100 heran geführt wird.

Es sind natürlich auch andere Anordnungen der Elektroden 3, 4, 5 möglich. Beispielsweise zeigt Fig. 3d eine Anordnung, in der der Aktivitätsbereich 6 auf den Bereich beschränkt ist, in welchem sich die zwei Elektroden 3, 4 am nächsten kommen. Dies ist beispielsweise für sehr selektive Geräte vorteilhaft, bei denen der Chirurg durch Drehen des Koagulations- und Schneidegerätes 1 den Ort der Koagulation genau bestimmen kann. Bei anderen Anordnungen können zusätzlich zu den zwei Elektroden 3, 4 auch weitere Elektroden 5 vorgesehen sein, wodurch der Koagulationsbereich verändert, insbesondere vergrößert, werden kann.

Die Elektroden 3, 4, 5 können bei dem vorgestellten Koagulations- und Schneidegerät 1 durch einen länglichen Grundkörper 2 getragen werden.

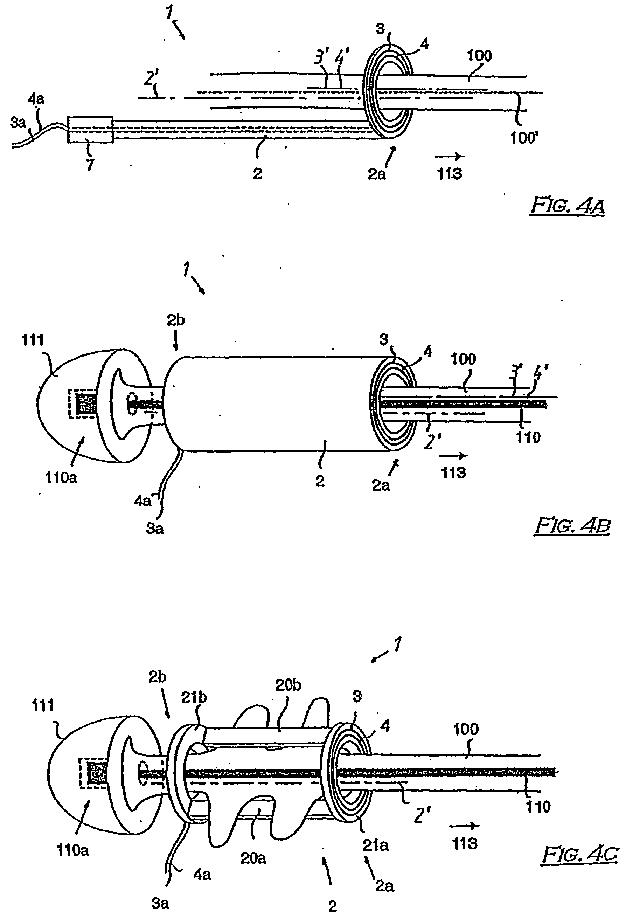

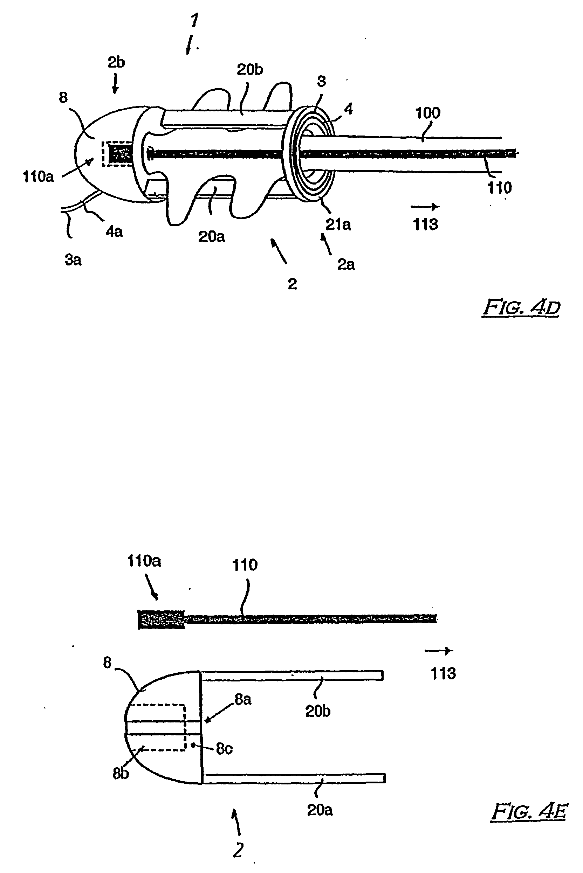

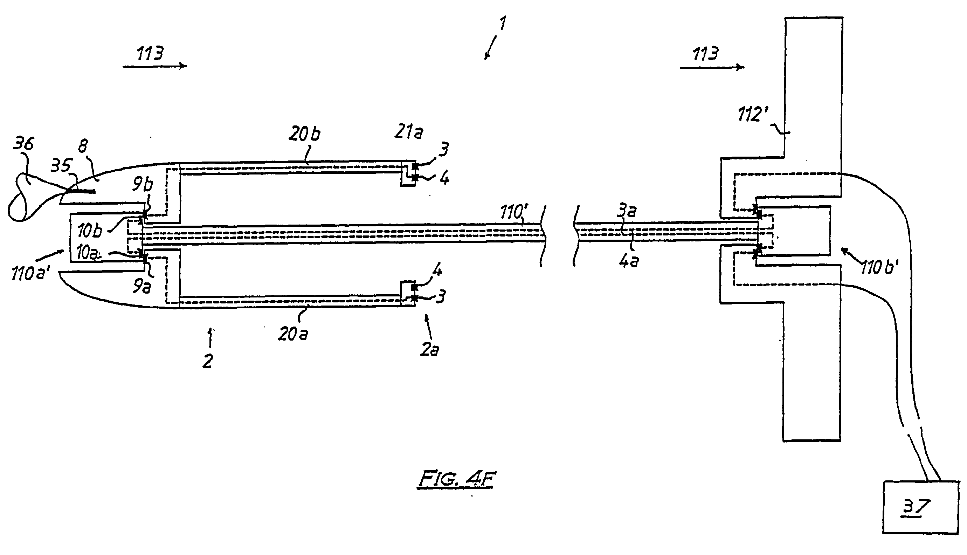

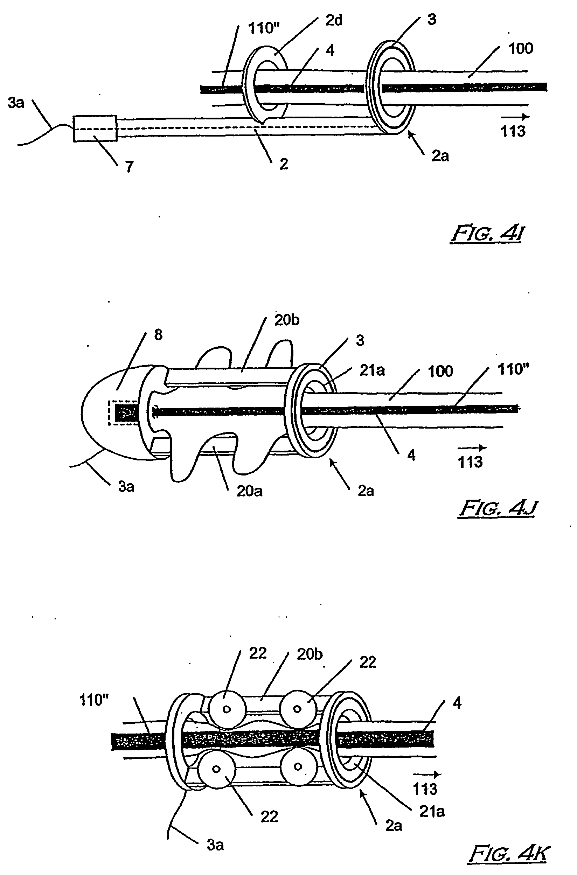

Die Fig. 4a bis 4k zeigen vorteilhafte Ausführungsformen des erfindungsgemäßen Venenstrippers. Dazu können die Elektroden 3, 4, 5 an der ersten Stirnseite 2a des Grundkörpers 2 so angebracht sein, daß die Symmetrieachsen 3',4' der Elektroden 3, 4, 5 im wesentlichen parallel zur Längsachse 2' des Grundkörpers 2 verläuft. Durch diese Anordnung wird gewährleistet, daß wenn der Grundkörper 2 in der dargestellten Weise bzw. in Zugrichtung 113 entlang der Vene 100 bzw. deren Mittellinie 100' bewegt wird, die Elektroden 3, 4, 5 in der oben beschriebenen Weise (insbesondere Fig. 3a-3d) gegenüber der Vene 100 ausgerichtet sind.

Der in Fig. 4a skizzierte Grundkörper 2 ist in Form eines einen Griff 7 aufweisenden Steges ausgebildet. Die Elektroden 3, 4, 5 liegen bei der dargestellten Ausführung, in Analogie zu der In Fig. 3c gezeigten Anordnung, in ein und derselben, im wesentlichen normal zur Längsachse 2' des Grundkörpers 2 verlaufenden Ebene. Es sind aber auch Anordnungen der Elektroden 3, 4, 5 gemäß der Fig. 3a, 3b, 3d od. dgl. möglich.

Die Elektroden 3, 4, 5 sind über elektrische Leitungen 3a, 4a mit einer Stromversorgungsquelle 37 verbunden, welche z.B. in Fig. 4f gezeigt ist.

Der in Fig. 4b skizzierte Grundkörper 2 weist eine in etwa zylindrische Form auf. An der ersten Stirnseite 2a des Grundkörpers 2 sind ringförmige Elektroden 3, 4, 5 angebracht, welche wieder über elektrische Leitungen 3a, 4a mit der Stromversorgungsquelle 37 verbunden sind. Auch hier verlaufen die Symmetrieachsen 3',4' der Elektroden 3, 4, 5 im wesentlichen parallel zur Längsachse 2' des Grundkörpers 2.

Durch die zylindrische Form ist es möglich, das Koagulations- und Schneidegerät 1 über die Vene 100 zu stülpen. Die zylindrische Form des in Fig. 4b dargestellten Grundkörpers 2 ermöglicht es weiters, daß das Koagulations- und Schneidegerät 1 entlang der Vene 100 geführt werden kann.

Gleichzeitig ist der Grundkörper 2 an seiner zweiten Stirnseite 2b so ausgestaltet, daß er auf einem gängigen Abschluß 111 eines Venenstrippers aufgesetzt werden kann.

Beim Venenstripping kann somit nach dem Überstülpen des erfindungsgemäßen Koagulations- und Schneidegeräts 1 auf das freigelegte Ende des zu entfernenden Abschnitts 102 der Vene 100 im Bereich 103 (vgl. Fig. 1b) und nach dem Einfädeln der Sonde 110 in die Vene 100 in bekannter Weise ein Abschluß 111 auf die Sonde 110 angebracht werden und die Vene in Zugrichtung 113 herausgezogen werden.

Bei diesem Vorgang werden jedoch im Unterschied zum bekannten Verfahren durch das erfindungsgemäßen Koagulations- und Schneidegerät 1 sämtliche venösen Seitenäste 101 abgeschnitten bzw. koaguliert, bevor diese mit den oben beschriebenen Nachteilen abreißen können.

Besonders vorteilhaft bei der beschriebenen Ausführung ist der Umstand, daß das Koagulations- und Schneidegerät 1 mit gängigen Venenstrippern verwendet werden kann.

Fig. 4c zeigt eine weitere Ausführungsform, bei der der Grundkörper 2 lediglich mittels zweier Stege 20a, 20b und der stirnseitigen Ringe 21a, 21b ausgebildet ist. Zweckmäßigerweise sind die elektrischen Leitungen 3a, 4a über die Stege 20a, 20b geführt. Es ist natürlich auch eine Ausführung mit lediglich einem Steg 20a möglich.

Durch diese Form des Grundkörpers 2 erreicht man, daß die durch den Grundkörper 2 aufgespannte im wesentlichen zylindrische Hülle im Unterschied zu der in Fig. 3b dargestellten Ausführung beinahe zur Gänze durchlässig ist.

Dies ist beim Venenstripping insbesondere im Zusammenhang mit der weiter oben beschriebenen Faltenbildung in der Venenwand vorteilhaft. Das Herausziehen der Vene 100 aus dem Bindegewebe 105 führt in dem dem Abschluß 111 vorgelagerten Bereich 114 regelmäßig zu einer Aufwölbung der Vene 100. Die in Fig. 4c dargestellte Ausführungsform erlaubt es, daß sich die Vene 100 genügend und über den durch den Grundkörper 2 umgrenzten zylindrischen Raum ausbreiten kann. Im Vergleich zur Ausführung mit einem geschlossenen Zylinder erlaubt es dieser Aufbau sehr viel kürzere Grundkörper 2 vorzusehen, ohne daß die Aufwölbung der Vene 100 zu einem Verstopfen des Grundkörpers 2 führt.

Auch hier können gängige Venenstripper verwendet werden.

Es sind natürlich auch Zwischenformen möglich, die konstruktiv zwischen den in Fig. 4b und 4c dargestellten Ausführungen liegen. Beispielsweise ist es möglich, lediglich einige größere Ausnehmungen in der zylindrischen Mantelfläche des Grundkörpers 2 vorzusehen. Auf diese Weise kann das Koagulations- und Schneidegerät 1 hinsichtlich Länge und Steifigkeit optimiert werden. Eine notwendige Voraussetzung für die Verwendung des Koagulationsund Schneidegerätes 1 für das Venenstripping ist lediglich, daß die Aufwölbung der Vene 100 im Bereich 114 sich nicht in Zugrichtung 113 vor die Elektroden 3, 4, 5 erstreckt. In diesem Fall könnten die venösen Seitenäste 101 nicht mehr sicher koaguliert bzw. geschnitten werden.

Bei der Darstellung in Fig. 4d ist an der zweiten Stirnseite 2b des Grundkörpers 2 eine Haltevorrichtung 8 angebracht. Diese dient zur Aufnahme und Fixierung des Endes 110a einer Sonde 110. Somit ist bei dieser Ausführung der Abschluß 111 im Grundkörper 2 integriert. Dadurch wird die Handhabung noch etwas vereinfacht, da kein eigener Abschluß 111 angebracht werden muß. Nach Einfädeln der Sonde 110 durch die freigelegte Vene 100 wird lediglich das erfindungsgemäße Koagulations- und Schneidegerät 1 über die Vene 100 gestülpt und das Ende 110a der Sonde 110 am Grundkörper 2 bzw. an der Haltevorrichtung 8 fixiert.

Die Haltevorrichtung 8 ist dazu vorzugsweise ähnlich ausgestaltet wie bekannte Abschlüsse 111 von gängigen Venenstrippern. Insbesondere weist sie einen Schlitz 8a auf, durch welchen die Sonde 110 eingelegt werden kann, weiters eine Vertiefung 8b zur Aufnahme des Endes 110a der Sonde 110 und einen Steg 8c, welcher ein Durchrutschen des Endes 110a verhindert (Fig. 4e).

Die skizzierte Ausführungsform erleichtert die Bedienung beim Venenstrippen, insbesondere kann verhindert werden, daß der Grundkörper 2 in der falschen Richtung über die Vene 100 gestülpt wird.

Bei der in Fig. 4f gezeigten Ausführungsform erfolgt die Verbindung der Elektroden 3, 4, 5 mit der Stromversorgungsquelle 37 über die Sonde 110. Damit ist kein eigenes Kabel notwendig, welches entweder vor dem Venenstripping durch die Vene 100 geführt oder beim Herausreißen der Vene 100 durch das Bindegewebe 105 mitgezogen werden muß.

Hierfür kann vorgesehen sein, daß die Haltevorrichtung 8 zwei Kontakte 9a 9b aufweist, die mit den Elektroden 3, 4, 5 elektrisch leitend verbunden sind. Weiters ist eine spezielle Sonde 110' vorgesehen, in welcher zwei elektrische Leitungen 3a und 4a eingeschmolzen sind. Die Sonde 110' weist an ihrem Ende 110a' ebenfalls zwei Kontakte 10a, 10b auf. Nach dem Durchfädeln der Sonde 110' durch die Vene 100 wird das Koagulations- und Schneidegerät 1 in ähnlicher Weise wie bei der in Fig. 4d gezeigten Ausführung mit dem Ende 110a' der Sonde 110' verbunden. Dabei erfolgt jetzt aber eine Kontaktierung zwischen den Kontakten 9a, 9b und 10a, 10b, wodurch die ringförmigen Elektroden 3, 4, 5 über die Sonde 110' mit der Stromversorgungsquelle 37 verbunden sind.

Vorzugsweise ist die Sonde 110' an beiden Enden 110a' und 110b' mit Elektroden 10a, 10b versehen und weiters ein eigener Griff 112' vorgesehen, welcher in analoger Weise eine elektrische Verbindung zu den elektrischen Leitern 3a, 4a herstellt. Der Griff 112' weist in diesem Fall ebenfalls Elektroden auf, die mit zu der Stromversorgungsquelle 37 führenden Leitern verbunden sind. In dieser letzten beschriebenen Ausführung gestaltet sich das Venenstripping besonders einfach. Nach Durchfädeln der Sonde 110' durch die Vene 100 und nach Aufsetzen des Koagulations- und Schneidegerätes 1 und des mit der Stromversorgungsquelle 37 verbundenen Griffs 112' an beiden Enden 110a' und 110b' der Sonde 110' sind die Elektroden 3, 4, 5 über den Griff 112' und die Sonde 110' mit der Stromversorgungsquelle 37 verbunden. Somit kann die Vene 100 herausgezogen werden, wobei alle venösen Seitenäste 101 mit großer Sicherheit durch die Elektroden 3, 4, 5 durchtrennt werden.

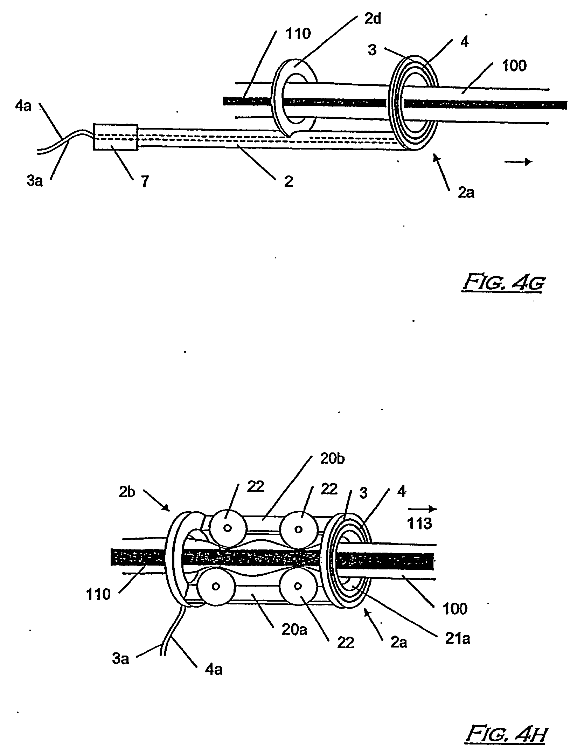

Der erfindungsgemäße Venenstripper gemäß Fig. 4g entspricht dem in Fig. 4a gezeigten, wobei zur Führung des Koagulations- und Schneidegerätes 1 die Sonde 110 vor dem Strippen in die Vene 100 eingeführt wurde. Dadurch kann verhindert werden, daß die Vene 100 durchschnitten wird, sodaß der erfindungsgemäße Venenstripper gemäß Fig. 4g auch eine Sonde umfaßt. Die Führung des Koagulations- und Schneidegerätes 1 wird durch den Führungsring 2d sichergestellt, wodurch gewährleistet wird, daß das Koagulations- und Schneidegerät 1 um die Vene geführt wird.

In Fig. 4h ist ein erfindungsgemäßer Venenstripper dargestellt, der eine Antriebsvorrichtung 22 aufweist. Diese kann insbesondere Räder 22 od. dgl. umfassen. Aufgrund der Antriebsvorrichtung 22 kann sich das Koagulations- und Schneidegerät 1 entlang der Sonde 110 bewegen und so die Vene 100 vom Bindegewebe 105 und den venösen Seitenästen 101 trennen. Bei dieser Ausführungsform sind keine zusätzlichen Schub- und/oder Ziehvorrichtungen erforderlich.

Die erfindungsgemäßen Venenstripper nach den Fig. 4g und 4h weisen den Vorteil auf, daß die Vene 100 in einem ersten Arbeitsschritt von dem Bindegewebe 105 und den venösen Seitenästen 101 getrennt werden kann und erst in einem zweiten Arbeitsschritt die Vene 100 entfernt wird. Dadurch ist es nicht erforderlich, daß das Koagulations- und Schneidegerät 1 Bereiche für die Aufnahme der bereits gestrippten Vene 100 aufweist, da diese unverändert im Körper verbleibt, wodurch kleine Bauformen für den erfindungsgemäßen Venenstripper erzielt werden können.

Bei den erfindungsgemäßen Venenstrippern 1 gemäß den Fig. 4i bis 4k ist jeweils die Sonde 110" als Elektrode 4 ausgebildet. Dadurch reicht eine Elektrode 3 am Koagulations- und Schneidegerät 1 bereits zur Erreichung der Schneid- und Koagulationswirkung aus. Durch die im wesentliche ringförmige Ausbildung der Elektrode 3 am Koagulations- und Schneidegerät 1 wird sichergestellt, daß in alle Richtungen geschnitten und koaguliert werden kann. Zur Verbesserung der Eigenschaften des Koagulations- und Schneidegeräts 1 können auch bei den Ausführungsformen gemäß der Fig. 4i bis 4k weitere Elektroden 5 vorgesehen sein.

Bei den Ausführungsformen, bei denen die Sonde 110, 110', 110" gemeinsam mit dem Koagulations- und Schneidegerät 1 beim Strippen bewegt wird, kann die Sonde 110, 110', 110" mit dem Koagulations- und Schneidegerät 1 auch einstückig verbunden sein.

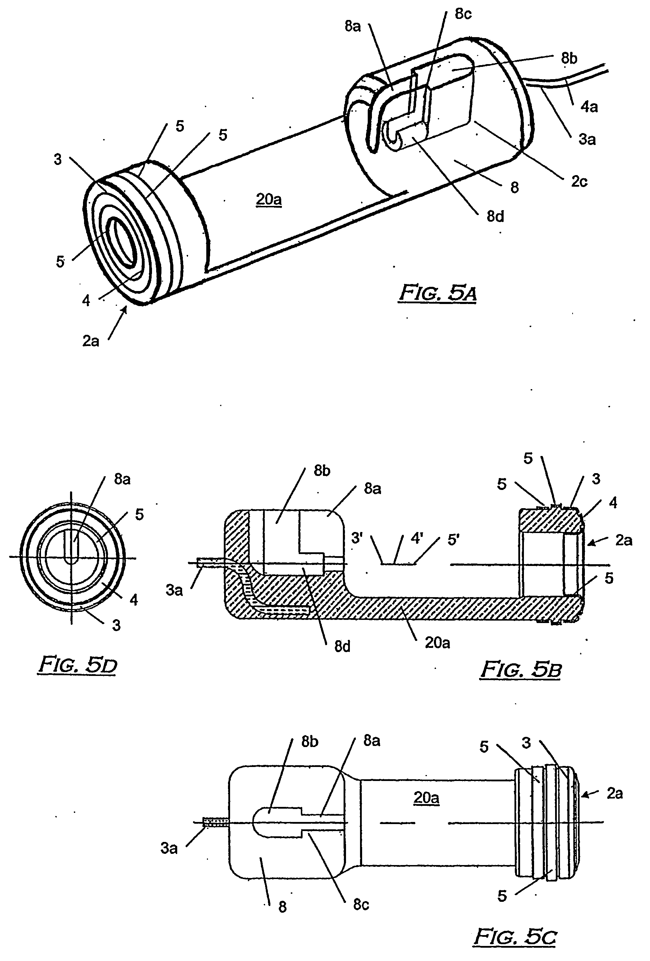

Es hat sich gezeigt, daß während der Operation der Venenstripper meist mehrfach ein Stückchen zurückgezogen werden muß. Dies kann durch Ziehen an den mit den Elektroden 3, 4, 5 verbundenen elektrischen Leitungen 3a, 4a bzw. an einem diese umschließenden Kabel erfolgen, wenn die Leitungen 3a, 4a bzw. das Kabel an der zweiten Stirnflächen 2b des Grundkörpers 2 herausgeführt sind bzw. ist. Ist das Kabel exzentrisch am Grundkörper 2 befestigt, so kann es zu einem Verkanten des Koagulations- und Schneidegerätes 1 im Kanal kommen. Die Leitungen 3a, 4a werden deshalb bei einer weiteren in den Fig. 5a bis 5d skizzierten vorteilhaften Ausführungsform zentrisch von der zweiten Stirnflächen 2b des Grundkörpers 2 weggeführt.

Das in den Fig. 5a bis 5d skizzierte Koagulations- und Schneidegerät 1 weist im Bereich der ersten Stirnfläche 2a des Grundkörpers 2 mehrere weitere Elektroden 5 auf, die sich im wesentlichen mit den Elektroden 3, 4 überdecken. Insbesondere verlaufen deren Symmetrieachsen 5' im wesentlichen parallel zu den Symmetrieachsen 3', 4' der anderen Elektroden 3,4. Im wesentlichen überlappen sich dabei bei Betrachtung in Achsrichtung der Symmetrieachsen 3', 4', 5' die von den Elektroden 3, 4, 5 aufgespannten Flächen.

Wie aus der Zeichnung ersichtlich ist, können die weiteren Elektroden 5 an der äußeren Wandfläche 2c des Grundkörpers 2 oder auch an der inneren Wandfläche angebracht werden. Natürlich ist auch das Vorsehen weiterer Elektroden unmittelbar auf der ersten Stirnfläche 2a des Grundkörpers 2 möglich.

Durch die Verwendung mehrerer Elektroden 3, 4, 5 entstehen weitere Aktivitätsbereiche 6, in denen die abgetrennten venösen Seitenäste 101 zusätzlich koaguliert werden können. Dies ist besonders vorteilhaft für die Blutstillung, da durch nochmaliges Koagulieren des bereits abgetrennten offenen Endes eines Venenastes eine bestmögliche Blutstillung erzielt werden kann. Dies ist oft bei sehr dicken oder auch bei sehr dünnen oder brüchigen Venenästen notwendig, bei denen ansonsten noch vereinzelte Blutungen auftreten können. Wird das Koagulations- und Schneidegerät 1 mit zusätzlichen Elektroden 5 an der Seitenfläche 2c des Grundkörpers 2 versehen, so werden die Enden der problematischen venösen Seitenäste 101 automatisch mehrfach koaguliert, wodurch eine vollständige Blutstillung erzielt werden kann.

Die zusätzlichen Ringelektroden 5 können über eigene Leitungen an ein bestimmtes Potential gelegt werden oder mit den anderen Elektroden 3, 4, 5 verbunden werden. Der erste Fall bietet die Möglichkeit, die Potentialdifferenz für alle Aktivitätsbereiche 6 getrennt einzustellen; im letzteren Fall können sämtliche Elektroden 3, 4, 5 vorteilhafterweise über die beiden Leitungen 3a, 4a versorgt werden. Die Elektroden 3, 4, 5 des in Fig. 5a bis 5d skizzierten Koagulations- und Schneidegerätes 1 sind alle über die Leitungen 3a, 4a verbunden und weisen abwechselnd die Polaritäten der Leitung 3a und der Leitung 4a auf.

Unterschiedliche Potentialdifferenzen zwischen den einzelnen Elektroden 3, 4, 5 kann auch durch unterschiedliche Abstände zwischen den Elektroden 3, 4, 5 und/oder durch die Verbindung zumindest einer der Elektroden 3, 4, 5 mit einem Widerstand od. dgl. erreicht werden. Als Widerstände kommen neben ohmschen Widerständen insbesondere Blindwiderstände in Betracht.

Der Grundkörper 2 ist mittels eines einzelnen breiten Steges 20a ausgeführt, wodurch genügend Platz für die Faltenbildung der Venenwand zur Verfügung steht.

Im Bereich der zweiten Stirnfläche 2b weist der Grundkörper 2 einen in der Seitenwand 2c vorgesehenen Schlitz 8a sowie eine Vertiefung 8b auf, wobei die Vertiefung 8b an Ihrer der ersten Stirnseite 2b des Grundkörpers 2 zugewandten Seite durch einen Steg 8c verengt ist. Dies dient als Haltevorrichtung 8 zur Aufnahme und Fixierung des Endes 110a einer Sonde 110. Die dargestellte Haltevorrichtung 8, bei der die Vertiefung 8b an Ihrer der ersten Stirnseite 2b des Grundkörpers 2 zugewandten Seite eine Verlängerung 8d aufweist, ermöglicht eine einfache formschlüssige Verbindung zwischen dem Grundkörper 2 und dem kopfartigen Ende 110a der Sonde 110. Nach dem Einlegen der Sonde 110 bzw. deren Endes 110a in den Bereich des Schlitzes 8a bzw. der Vertiefung 8b kann das Ende 110a der Sonde durch Zurückziehen in Zugrichtung 113 in der Verlängerung 8d festgelegt werden.

Durch das Vorsehen der Haltevorrichtung 8 in der Seitenwand 2c können die Leitungen 3a, 4a zentrisch von der zweiten Stirnfläche 2b weggeführt werden. Damit kann das Koagulations- und Schneidegerät 1 auf einfache Weise an den Leitungen 3a, 4a und an der Sonde 110 im Venenkanal hin- und hergezogen werden.

Insbesondere für den Fall, daß die Leitungen 3a, 4a innerhalb der Sonde 110' geführt sind, besteht die Möglichkeit, einen zusätzlichen Drainageschlauch 36 an der zweiten Stirnfläche 2b festzulegen, wie dies z.B. in Fig. 4f dargestellt ist. Dieser Drainageschlauch oder Redonschlauch 36 wird für gewöhnlich nach erfolgter Extraktion der Vene 100 in den Stripkanal eingebracht um zusätzliche Gewebsflüssigkeit aufzunehmen. Die Ausführung eines Koagulations- und Schneidegerätes 1 mit festgelegtem Redonschlauch 36 erleichtert einerseits das Einbringen dieses Schlauches 36 da der Redonschlauch 36 mit dem Entfernen der Vene 100 automatisch in den Venenkanal eingeführt wird. Andererseits kann für den Fall, daß die Leitungen 3a, 4a innerhalb der Sonde 110' geführt sind und der Grundkörper nicht mehr an den Leitungen 3a, 4a zurückgezogen werden kann, der erfindungsgemäße Venenstripper nunmehr am Redon- bzw. Drainageschlauch 36 im Venenkanal hin- und hergezogen werden. Die Festlegung des Drainageschlauches 36 kann auf unterschiedliche Arten beispielsweise durch Verschweißen oder Einklemmen erfolgen. Vorteilhafterweise ist aber eine eigene Haltevorrichtung 35 am Grundkörper 2 vorgesehen mittels derer der Drainageschlauch 36 festgelegt werden kann. Dies bietet die Möglichkeit, die Operation an einem Bein durchzuführen, den ersten Redonschlauch 36 im ersten Venenkanal zu belassen, einen weiteren Redonschlauch 36 mit der Haltevorrichtung 35 am Grundkörper 2 zu fixieren und die Operation am zweiten Bein zu wiederholen. Bei anderen Ausführungsformen des erfindungsgemäßen Venenstrippers können die Leitungen 3a, 4a innerhalb des Redonschlauches 36 geführt sein.

In Fig. 5e ist die Seitenansicht einer weiteren Ausführungsform des Koagulations- und Schneidegerätes 1 mit einer Längs-Schlitzvorrichtung 25, im Schnitt dargestellt. Diese umfaßt zusätzlich zu der Ausführungsform gemäß der Fig. 5a bis 5d die Längs-Schlitzvorrichtung 25.

Mit der Längs-Schlitzvorrichtung 25 wird die Vene 100 beim Strippen in Längsrichtung aufgeschlitzt, wobei der aufgeschlitzte Bereich der Vene 100 um die zweite Stirnseite 2b des Koagulations- und Schneidegerätes 1 geführt werden kann. Daher kann das Koagulationsund Schneidegerät 1 mit der Sonde 110, 110', 110" durch den Stripkanal gezogen werden, wobei die Vene 100 im Stripkanal verbleibt und erst in einem weiteren Arbeitsschritt aus dem Stripkanal entfernt wird. Bei dem Entfernen der Vene 100 kann ein Redonschlauch in den Stripkanal eingebracht werden. Bei dieser Vorgangsweise ist es nicht erforderlich, daß der erfindungsgemäße Venenstripper den bereits gestrippten Bereich der Vene 100 aufnehmen kann, wodurch kleinere Bauformen des erfindungsgemäßen Venenstrippers erreichbar sind.

Das Längsschlitzen der Vene 100 kann mittels einer mechanischen Schneide oder mittels einer Schlitzelektrode erfolgen, wobei mit der Schlitzelektrode bipolar oder monopolar geschnitten werden kann. Bei einer bipolaren Ausführung der Schlitzelektrode kann eine der weiteren Elektroden 5 als Gegenelektrode verwendet werden. Bei einer monopolaren Schlitzelektrode können die benötigten Leistungen für das Schneiden und Koagulieren der Vene 100 und das Längsschlitzen unabhängig voneinander eingestellt werden.

Bei der Ausführungsform gemäß Fig. 5e ist die Längs-Schlitzvorrichtung 25 mit dem Koagulations- und Schneidegerät 1 verbunden. Dadurch ist es möglich, die Schlitzelektrode mit einer der elektrischen Leitungen 3a, 4a zu verbinden. Insbesondere bei einer monopolaren Schlitzelektrode hat sich das Vorsehen einer eigenen elektrischen Leitung als günstig erwiesen.

Das Längsschlitzen der Vene 100 ist insbesondere dann sichergestellt, wenn die Längs-Schlitzvorrichtung 25 sowohl mit dem Koagulations- und Schneidegerät 1 als auch mit der Sonde 110, 110', 110" verbunden ist. Dazu kann vorgesehen sein, daß die Sonde 110, 110', 110" eine Nut od. dg1. aufweist, in die die Längs-Schlitzvorrichtung 25 verrastbar ist.

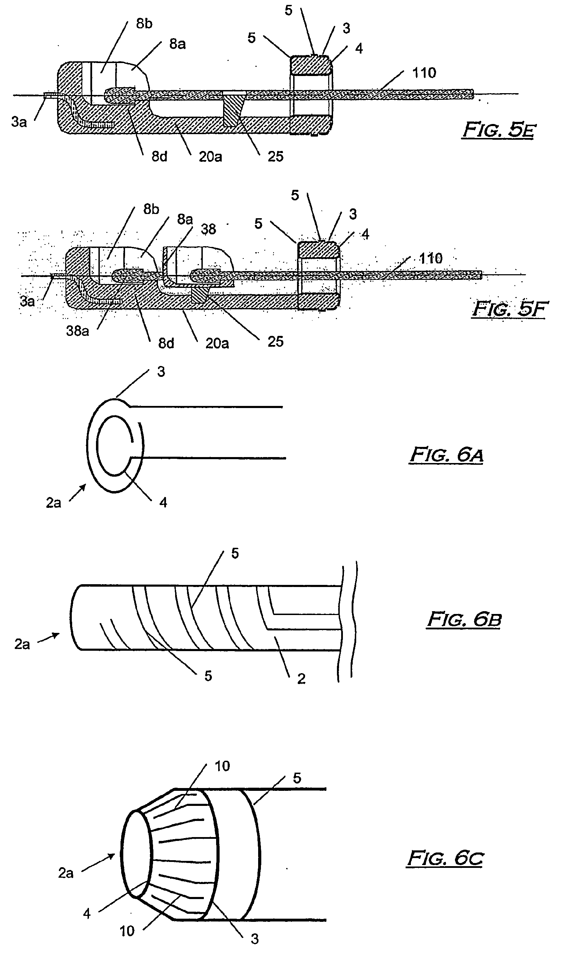

Gemäß einer anderen Ausführungsform des erfindungsgemäßen Venenstrippers, welche in Fig. 5f dargestellt ist, kann die Längs-Schlitzvorrichtung 25 in der Haltevorrichtung 8 mittels eines Fortsatzes 38a fixiert werden und eine weitere Haltevorrichtung 38 zur Aufnahme und Fixierung des Endes 110a, 110a', 110b' der Sonde 110, 110', 110" aufweisen. Bei dieser Ausführungsform kann die Längs-Schlitzvorrichtung 25 zwischen der Sonde 110, 110', 110" und der Haltevorrichtung 8 des Koagulations- und Schneidegerätes 1 eingebracht werden, wodurch sich ein modularer Aufbau ergibt. Auch diese Bauform der Längs-Schlitzvorrichtung 25 kann einstückig mit dem Koagulations- und Schneidegerät 1 verbunden sein.

Bei den gezeigten Ausführungsformen ergibt sich aufgrund der Anordnung der Elektroden 3, 4, 5, daß sich der Aktivitätsbereich 6 im wesentlichen über den gesamten Umfang des Grundkörpers erstreckt. Diese Eigenschaft ist auch mit einer Vielzahl weiterer Elektrodenformen erreichbar.

Beispielsweise können die Elektroden 3, 4, 5 abschnittsweise Unterbrechungen aufweisen, wie in Fig. 6a skizziert ist. Dies bietet die zusätzliche Möglichkeit, die Elektroden 3, 4, 5 spiralförmig zu verlängern. Fig. 6b zeigt ein Koagulations- und Schneidegerät 1, bei dem zwei der Elektroden 3, 4, 5 spiralförmig über einen längeren Bereich des Grundkörpers 2 verlängert sind. Damit erhält man einen größeren Aktivitätsbereich 6.

Bei der in Fig. 6c gezeigten Ausführung weisen die Elektroden 3, 4, 5 fächerförmige Finger 10 auf. Auch dadurch ergibt sich die Möglichkeit, den Aktivitätsbereich 6 zu vergrößern.

Neben dem Strippen von Venen 100 kann der erfindungsgemäße Venenstripper auch zum Strippen anderer schlauch- und/oder röhrenförmiger Gewebeteile od. dgl. verwendet werden. Beispielsweise könnte ein Bereich einer Speiseröhre mit dem erfindungsgemäßen Venenstripper gestrippt werden.

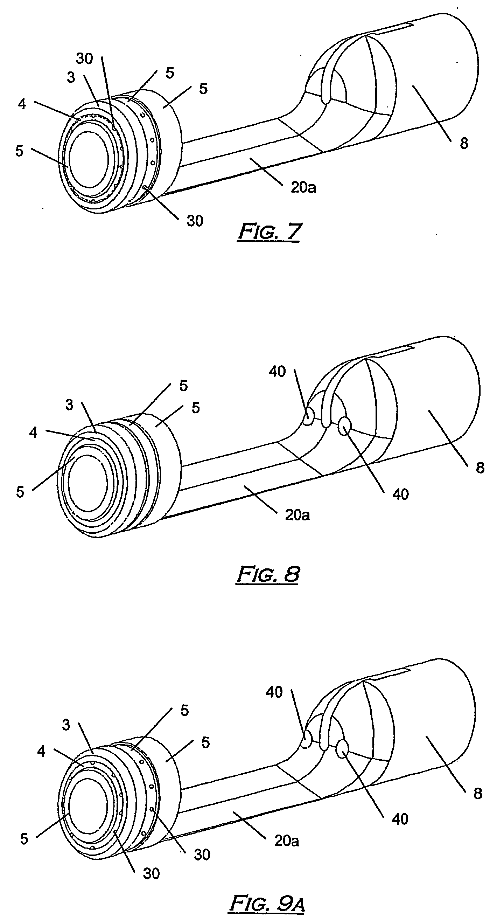

Der in Fig. 7 gezeigte erfindungsgemäße Venenstripper weist Auslaßöffnungen 30 für ein ionisierbares Gas auf, wobei als ionisierbares Gas insbesondere die Verwendung von Argon vorgesehen sein kann. Die Auslaßöffnungen 30 sind vorzugsweise um den gesamten Umfang des erfindungsgemäßen Venenstrippers angeordnet, wobei eine Vielzahl an Auslaßöffnungen 30 oder eine im wesentlichen ringförmige Auslaßöffnung vorgesehen sein kann. Es hat sich als günstig erwiesen, die Elektroden 3, 4, 5 mit derselben Polarität mit den Auslaßöffnungen 30 auszubilden, wobei benachbart zu den Elektroden 3, 4, 5 mit den Auslaßöffnungen 30 die Elektroden 3, 4, 5 der anderen Polarität angeordnet sind.

Bei einem erfindungsgemäßen Venenstipper kann es ohne der Verwendung von ionisierbarem Gas vorkommen, daß die koagulierte Kruste eines der Seitenäste an den Elektroden 3, 4, 5 verklebt und aufgrund der Bewegung des Venenstrippers die Kruste aufbricht und der Seitenast erneut zu bluten beginnt. Durch mehrmaliges Durchziehen des Venenstrippers kann auch ohne Verwendung von ionisierbaren Gas eine vollständige Koagulation der Seitenäste erreicht werden.

Bei der Verwendung des ionisierbaren Gases strömt dieses aus den Auslaßöffnungen 30 und bildet über der Elektrode 3, 4, 5 mit den Auslaßöffnungen 30 ein Gaspolster. Berührt der Gaspolster eine der Elektroden 3, 4, 5 mit einer anderen Polarität, so ionisiert das Gas und wird leitend, wobei sich das Gas erhitzt und plasmaförmig wird. Das den Gaspolster berührende Gewebe wird von diesem koaguliert, wobei ein direkter Kontakt des Gewebes mit einer der Elektroden 3, 4, 5 nicht auftritt und daher auch keine Krusten an den Elektroden 3, 4, 5 verkleben können.

Um sicherzustellen, daß durch die Hitze des plasmaförmigen ionisierten Gases, und nicht direkt auf den Elektroden 3, 4, 5, koaguliert wird, können die Elektroden 3, 4, 5 mit den Auslaßöffnungen 30 gegenüber den benachbarten Elektroden 3, 4, 5 zurückversetzt sein, wodurch die Bildung des Gaspolsters aus plasmaförmigen Gas über den Elektroden 3, 4, 5 mit den Auslaßöffnungen 30 erreicht wird. Dieser Gaspolster, welcher insbesondere ringförmig sein kann, bewirkt die Koagulation und verhindert gleichzeitig den direkten Kontakt des zu koagulierenden Gewebes mit den Elektroden 3, 4, 5.

Weist der Gaspolster nur zu einer der Elektroden 3, 4, 5 direkten Kontakt auf, so kann der Kontakt zu einer der Elektroden 3, 4, 5 mit einer anderen Polarität auch indirekt über das Gewebe erfolgen. Dabei ionisiert das Gas zwischen der Elektrode 3, 4, 5 mit den Auslaßöffnungen 30 und dem Körpergewebe, wobei sich das Gas wieder erhitzt und plasmaförmig wird, wodurch das Gewebe koaguliert wird. Dieser Fall kann insbesondere dann auftreten, wenn der Gasdruck sehr gering ist, die Ränder der Nachbarelektroden verschmutzt sind und/oder sehr viel Körperflüssigkeit an den Oberflächen der Elektroden 3, 4, 5 auftritt.

Sind die Elektroden 3, 4, 5 nur geringfügig zurückversetzt, kann sichergestellt werden, daß im Falle des Ausfalls des Gasstromes das zu koagulierende Gewebe direkt mit den Elektroden 3, 4, 5 in Berührung kommt, und eine Koagulation wie bei einem erfindungsgemäßen Venenstripper ohne Unterstützung mit ionisierbarem Gas erfolgt.

Der erfindungsgemäße Venenstripper gemäß Fig. 8 weist im Bereich der Haltevorrichtung 8 zwei Absaugöffnungen 40 auf, mittels denen der beim Koagulieren entstehende Rauch abgesaugt werden kann. In anderen Ausführungsformen kann auch eine andere Anzahl an Absaugöffnungen 40 vorgesehen sein. Die Anordnung der Absaugöffnungen 40 im Bereich der Haltevorrichtung 8 hat den Vorteil, daß die Ausgestaltung der Elektroden 3, 4, 5 unabhängig von der Ausgestaltung der Absaugöffnungen 40 erfolgen kann. Durch die Absaugöffnungen 40 wird das unmittelbare Absaugen des Rauches nach seinem Entstehen ermöglicht.

Die Absaugöffnungen 40 können auch in dem Steg 20a angeordnet sein.

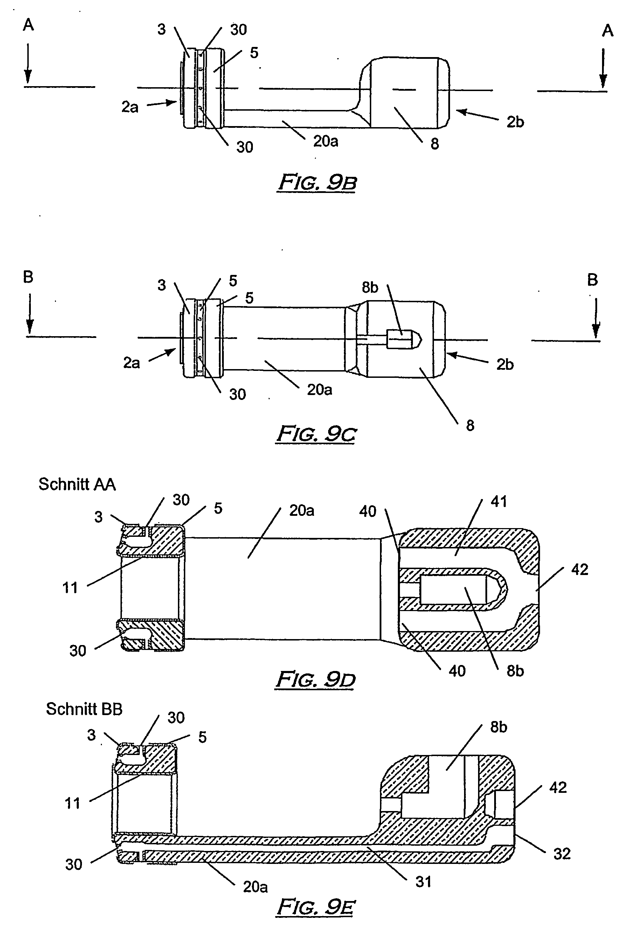

In den Fig. 9a bis 9e ist eine weitere Ausführungsform des erfindungsgemäßen Venenstrippers dargestellt, welche Auslaßöffnungen 30 und Absaugöffnungen 40 aufweist.

Aus den in den Fig. 9d und 9e gezeigten Schnitten ist ersichtlich, daß eine der Elektroden 3, 4, 5 einen rohrförmigen Bereich 11 aufweist. Der rohrförmige Bereich weist eine große Kontaktfläche auf, wodurch die leitende Verbindung zwischen dem rohrförmigen Bereich 11 und dem Gewebe verbessert wird.

Weiters geht aus den Fig. 9d und 9e hervor, daß die Auslaßöffnungen 30 mittels einer Gasleitung 31 mit einem Gasanschluß 32 verbunden sind. Der Gasanschluß 32 kann an der zweiten Stirnseite 2b des Grundkörpers 2 angeordnet sein. Dann kann die Gaszuleitung parallel zu den elektrischen Leitungen 3a, 4a geführt oder mit diesen verbunden werden.

Die Absaugöffnungen 40 sind mittels Absaugbohrungen 41 mit einem Absauganschluß 42 verbunden, welcher vorzugsweise ebenfalls an der zweiten Stirnseite 2b des Grundkörpers 2 angeordnet ist. Dann kann auch eine Absaugleitung parallel zu den elektrischen Leitungen 3a, 4a geführt oder mit diesen verbunden werden. Die elektrischen Leitungen 3a, 4a, die Gaszuleitung und die Absaugleitung können auch in einem gemeinsamen Schlauch od. dgl. geführt werden.

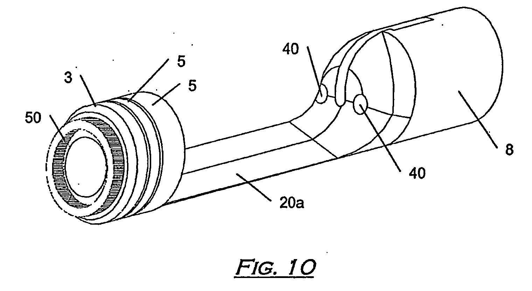

Bei der in Fig. 10 dargestellten Ausführungsform des erfindungsgemäßen Venenstrippers ist im Bereich der Elektroden 3, 4, 5 ein ringförmiger Ultraschallresonator 50 angeordnet, welcher als Schneidegerät ausgebildet ist. Die Elektroden 3, 4, 5 bilden das Koagulationsgerät. Bei dieser Ausbildung sind der Schneidevorgang und der Koagulationsvorgang unterschiedlich realisiert, wodurch auch eine getrennte Regelung der beiden Vorgänge auf einfache Weise möglich ist.

Bei den Ausführungsformen gemäß den Fig. 7 bis 10 kann auch die Längs-Schlitzvorrichtung 25 vorgesehen sein. Weiters können der Ultraschallresonator 50 und/oder die Absaugöffnungen 40 und/oder die Auslaßöffnungen 30 bei allen Ausführungsformen vorgesehen sein.

## Patentansprüche

1. Venenstripper mit einer Sonde und einem Koagulations- und Schneidegerät, wobei das Koagulations- und Schneidegerät zumindest zwei Elektroden und eine stirnseitige Öffnung, durch die die Sonde führbar ist, aufweist, **dadurch gekennzeichnet, daß** über den im Bereich der stirnseitigen Öffnung gelegene Umfang des Koagulations- und Schneidegerätes (1) wenigstens zwei der zumindest zwei Elektroden (3,4,5) benachbart angeordnet sind.

2. Venenstripper mit einer Sonde und einem Koagulations- und Schneidegerät, wobei das Koagulations- und Schneidegerät zumindest eine Elektrode und eine stirnseitige Öffnung, durch die die Sonde führbar ist, aufweist, **dadurch gekennzeichnet, daß** die Sonde (110,110',110") zumindest entlang eines Bereiches ihrer Länge als Elektrode (3,4,5) ausgebildet ist.

3. Venenstripper nach Anspruch 2, **dadurch gekennzeichnet, daß** über den im Bereich der stirnseitigen Öffnung gelegene Umfang des Koagulations- und Schneidegerätes (1) wenigstens eine der zumindest einen Elektrode (3,4,5) des Koagulations- und Schneide gerätes (1) angeordnet ist.

4. Venenstripper nach einem der Ansprüche 1 bis 3, **dadurch gekennzeichnet, daß** wenigstens eine der Elektroden (3,4,5) ringförmig ausgebildet ist.

5. Venenstripper nach einem der Ansprüche 1 bis 4, **dadurch gekennzeichnet, daß** wenigstens eine der Elektroden (3,4,5) spiralförmig ausgebildet ist.

6. Venenstripper nach einem der Ansprüche 1 bis 5, **dadurch gekennzeichnet, daß** wenigstens eine der Elektroden (3,4,5) Unterbrechungen aufweist.

7. Venenstripper nach einem der Ansprüche 1 bis 6, **dadurch gekennzeichnet, daß** wenigstens eine der Elektroden (3,4,5) fächerförmige Finger (10) aufweist.

8. Venenstripper nach einem der Ansprüche 1 bis 7, **dadurch gekennzeichnet, daß** ein Ende (110a,110a',110b') der Sonde (110,110',110") mit dem Koagulations- und Schneidegerät (1) mittels einer am Koagulations- und Schneidegerät (1) angeordneten Haltevorrichtung (8) oder mittels eines Abschlusses (111) od. dgl. verbindbar ist.

9. Venenstripper nach Anspruch 8, **dadurch gekennzeichnet, daß** die Sonde (110,110',110") mit dem Koagulations- und Schneidegerät (1) fixiert ist.

10. Venenstripper nach einem der Ansprüche 1 bis 9, **dadurch gekennzeichnet, daß** das Koagulations- und Schneidegerät (1) eine Verschiebeeinrichtung, insbesondere einen Stiel (2) od. dgl., aufweist und daß die Sonde als Führungssonde (110,110',110") ausgebildet ist.

11. Venenstripper nach einem der Ansprüche 1 bis 10, **dadurch gekennzeichnet, daß** das Koagulations- und Schneidegerät (1) eine Antriebsvorrichtung (22) aufweist, wobei es selbsttätig entlang der Sonde (110,110',110") bewegbar ist.

12. Venenstripper nach einem der Ansprüche 1 bis 11, **dadurch gekennzeichnet, daß** das Koagulations- und Schneidegerät (1) zumindest eine weitere Elektrode (5), insbesondere zumindest drei weitere Elektroden (5), umfaßt.

13. Venenstripper nach Anspruch 12, **dadurch gekennzeichnet, daß** die zumindest eine weitere Elektrode (5) an der Wandfläche (2c) des Koagulations- und Schneidegerätes (1) angeordnet ist.

14. Venenstripper nach Anspruch 12 oder 13, **dadurch gekennzeichnet, daß** die Elektroden (3,4,5) mit unterschiedlichen Abständen zu den jeweils benachbarten Elektroden (3,4,5) angeordnet sind.

15. Venenstripper nach einem der vorhergehenden Ansprüche, **dadurch gekennzeichnet, daß** zumindest eine der Elektroden (3,4,5) mit einem Widerstand od. dgl. verbunden ist.

16. Venenstripper nach einem der Ansprüche 1 bis 15, **dadurch gekennzeichnet, daß** das Koagulations- und Schneidegerät (1) einen entlang einer Längsachse (2') ausgerichteten Grundkörper (2) aufweist und die wenigstens zwei der zumindest zwei Elektroden (3,4,5) an einer ersten Stirnseite (2a) des Grundkörpers (2) angebracht sind.

17. Venenstripper nach einem der Ansprüche 1 bis 16, **dadurch gekennzeichnet, daß** das Koagulations- und Schneidegerät (1) zwei im wesentlichen ringförmig ausgebildete Elektroden (3,4) aufweist, wobei die Symmetrieachsen (3',4') der ringförmigen Elektroden (3,4) im wesentlichen parallel zueinander verlaufen und sich die von den ringförmigen Elektroden (3,4) jeweils aufgespannten Flächen bei Betrachtung in Achsrichtung der Symmetrieachsen (3',4') im wesentlichen überlappen.

18. Venenstripper nach Anspruch 17, **dadurch gekennzeichnet, daß** die zumindest eine weitere Elektrode (5) ringförmig ausgebildet ist, deren Symmetrieachse (5') im wesentlichen parallel zu den Symmetrieachsen (3',4') der beiden anderen ringförmigen Elektroden (3,4) verläuft, wobei sich die von der zumindest einen weiteren ringförmigen Elektrode (5) aufgespannte Fläche und die von den beiden anderen ringförmigen Elektroden (3,4) aufgespannten Flächen bei Betrachtung in Achsrichtung der Symmetrieachsen (3',4',5') im wesentlich überlappen.

19. Venenstripper nach Anspruch 17 oder 18, **dadurch gekennzeichnet, daß** die Symmetrieachsen (3',4') der ringförmigen Elektroden (3,4) im wesentlichen parallel zur Längsachse (2') des Grundkörpers (2) verlaufen.

20. Venenstripper nach Anspruch 17, 18 oder 19, **dadurch gekennzeichnet, daß** die ringförmigen Elektroden (3,4) in ein und derselben, im wesentlichen normal zur Längsachse (2') des Grundkörpers (2) verlaufenden Ebene liegen.

21. Venenstripper nach einem der Ansprüche 16 bis 20, **dadurch gekennzeichnet, daß** der Grundkörper (2) einen im wesentlichen zylindrischen Raum umgrenzt.

22. Venenstripper nach einem der Ansprüche 16 bis 21, **dadurch gekennzeichnet, daß** der Grundkörper (2) mit zumindest einem in Längsachse (2') des Grundkörpers (2) ausgerichteten Steg (20a) ausgebildet ist.

23. Venenstripper nach einem der Ansprüche 16 bis 22, **dadurch gekennzeichnet, daß** der Grundkörper (2) im Bereich seiner zweiten Stirnseite (2b) die Haltevorrichtung (8) zur Aufnahme und Fixierung des Endes (110,110a',110b') der Sonde (110,110',110") aufweist.

24. Venenstripper nach Anspruch 23, **dadurch gekennzeichnet, daß** der Grundkörper (2) an seiner zweiten Stirnseite (2b) die Haltevorrichtung (8) zur Aufnahme und Fixierung des Endes (110,110a',110b') der Sonde (110,110',110") aufweist.

25. Venenstripper nach einem der Ansprüche 8 bis 24, **dadurch gekennzeichnet, daß** die Haltevorrichtung (8) eine formschlüssige Verbindung zwischen dem Ende (110,110a',110b') der Sonde (110,110',110") und dem Grundkörper (2) ermöglicht.

26. Venenstripper nach einem der Ansprüche 16 bis 25, **dadurch gekennzeichnet, daß** die Haltevorrichtung (8) einen in der Seitenwand (2c) des Grundkörpers (2) angebrachten Schlitz (8a) zum Einlegen der Sonde (110,110',110") sowie eine Vertiefung (8b) zur Aufnahme des Endes (110a,110a',110b') der Sonde (110,110',110") umfaßt, wobei die Vertiefung (8b) an Ihrer der ersten Stirnseite (2a) des Grundkörpers (2) zugewandten Seite einen diese verengenden Steg (8c) aufweist.

27. Venenstripper nach Anspruch 26, **dadurch gekennzeichnet, daß** die Vertiefung (8b) an Ihrer der ersten Stirnseite (2a) des Grundkörpers (2) zugewandten Seite eine Verlängerung (8d) aufweist.

28. Venenstripper nach einem der Ansprüche 16 bis 27, **dadurch gekennzeichnet, daß** elektrische Leitungen (3a,4a) zur Verbindung der Elektroden (3,4,5) mit einer Stromversorgungsquelle (37) zentrisch an der zweiten Stirnseite (2b) des Grundkörpers (2) aus dem Grundkörper herausgeführt sind.

29. Venenstripper nach einem der Ansprüche 8 bis 28, **dadurch gekennzeichnet, daß** die Haltevorrichtung (8) zwei Kontakte (9a,9b) aufweist, die mit den Elektroden (3,4,5) elektrisch leitend verbunden sind und daß die elektrischen Leitungen (3a,4a) zur Verbindung der Elektroden (3,4,5) mit einer Stromversorgungsquelle (37) innerhalb der Sonde (110,110',110") geführt und mit an dem Ende (110a,110a',110b') der Sonde (110,110',110") liegenden weiteren Kontakten (10a,10b) verbunden sind, wobei die elektrisch leitende Verbindung zwischen den ringförmigen Elektroden (3,4,5) und der Stromversorgungsquelle (37) bei der Aufnahme und Fixierung der Sonde (110,110',110") in der Haltevorrichtung (8) durch die Kontakte (9a,9b) und die weiteren Kontakte (10a, 10b) erfolgt.

30. Venenstripper nach einem der vorhergehenden Ansprüche, **dadurch gekennzeichnet, daß** ein Drainageschlauch am Koagulations- und Schneidegerät (1) angeordnet ist.

31. Venenstripper nach Anspruch 30, **dadurch gekennzeichnet, daß** an der der Öffnung gegenüberliegenden Stirnseite (2b) des Koagulations- und Schneidegerätes (1) eine Drainageschlauch-Befestigungsvorrichtung angeordnet ist.

32. Venenstripper nach einem der Ansprüche 1 bis 31, **dadurch gekennzeichnet, daß** eine Längs-Schlitzvorrichtung (25) vorgesehen ist.

33. Venenstripper nach Anspruch 32, **dadurch gekennzeichnet, daß** die Längs-Schlitzvorrichtung (25) eine Schlitzelektrode zum monopolaren Schneiden, eine Schlitzelektrode zum bipolaren Schneiden oder eine mechanische Schneide umfaßt.

34. Venenstripper nach Anspruch 32 oder 33, **dadurch gekennzeichnet, daß** die Längs-Schlitzvorrichtung (25) mit dem Koagulations- und Schneidegerät (1) verbunden ist, und daß gegebenenfalls die Schlitzelektrode mit den elektrischen Leitungen (3a,4a) oder einer weiteren elektrischen Leitung verbunden ist.

35. Venenstripper nach Anspruch 34, **dadurch gekennzeichnet, daß** die Längs-Schlitzvorrichtung (25) mit der Sonde (110,110',110"), insbesondere mit einer Nut od. dgl. der Sonde (110,110',110"), verrastbar ist.

36. Venenstripper nach Anspruch 32 oder 33, **dadurch gekennzeichnet, daß** die Längs-Schlitzvorrichtung (25) in der Haltevorrichtung (8) fixiert werden kann und eine weitere Haltevorrichtung (38) zur Aufnahme und Fixierung des Endes (110a,110a',110b') der Sonde (110,110',110") aufweist.

37. Venenstripper nach einem der Ansprüche 1 bis 36, **dadurch gekennzeichnet, daß** im Bereich wenigstens einer der Elektroden (3, 4, 5) wenigstens eine Auslaßöffnung (30) für den Austritt eines ionisierbaren Gases, insbesondere Argon, vorgesehen ist.

38. Venenstripper nach Anspruch 37, **dadurch gekennzeichnet, daß** die wenigstens eine Auslaßöffnung (30) mit einem Gasanschluß (32) an der zweiten Stirnseite (2b) des Grundkörpers (2) verbunden ist.

39. Venenstripper nach einem der Ansprüche 1 bis 38, **dadurch gekennzeichnet, daß** zumindest eine Absaugöffnung (40) vorgesehen ist.

40. Venenstripper nach Anspruch 39, **dadurch gekennzeichnet, daß** die zumindest eine Absaugöffnung (40) in der Haltevorrichtung (8) angeordnet ist.

41. Venenstripper nach Anspruch 39 oder 40, **dadurch gekennzeichnet, daß** die zumindest eine Absaugöffnung (40) mit einem Absauganschluß (42) an der zweiten Stirnseite (2b) des Grundkörpers (2) verbunden ist.

42. Venenstripper nach einem der Ansprüche 1 bis 41, **dadurch gekennzeichnet, daß** im Bereich der Elektroden (3, 4, 5) ein ringförmiger Ultraschallresonator (50) angeordnet ist.
